# EUROPEAN PATENT APPLICATION

(11) **EP 2 194 382 A1**
(43) Date of publication of application: **09.06.2010**
(21) Application number: 08170564.2
(22) Date of filing: 03.12.2008
(51) Int. Cl.: G01N 33/574

(54) **An in-vitro method or assay for detecting a tumor or a cancerous disease by screening for antibodies**

(71) Applicant: Universität des Saarlandes, 66123 Saarbrücken (DE)
(72) Inventor: Meese, Eckart, 66882 Hütschenhausen (DE); Lenhof, Hans-Peter, 66822 Lebach (DE)
(74) Representative: Polypatent

(57) **Abstract**

The present invention refers to an in-vitro method or assay for detecting whether or not a human individual has a tumor or a cancerous disease, comprising screening for the presence of antibodies in a blood or serum sample of said human individual, wherein the antibodies are directed to the antigens enolase 1 and latrophilin 1. In preferred embodiments, for the presence of antibodies directed to further antigens is screened additionally.

## Description

The present invention refers to a method and assay for diagnosing a tumor disease or cancerous disease outside of a living body, comprising screening for the presence of antibodies in a blood or serum sample of said human individual, wherein the antibodies are directed to specific antigens representing tumor markers.

One of the most desired goals in human cancer research is the identification of biomarkers to improve diagnosis of cancer, especially while the tumor is still in its early stages of development. Despite the progress in cancer diagnosis the timely detection of many cancer types is still a grand challenge. Tumor antigens offer themselves as tumor markers for cancer detection. Recently, it has been shown that antibody signatures of cancer patients' sera allow detection of various cancers. For various human cancer types including lung cancer, prostate cancer, and breast cancer, it was recently demonstrated that autoantibody profiling might be a promising approach towards earlier and more accurate cancer diagnosis.

Tumor markers have been successfully applied to detect various cancer types. Popular examples are the Prostate Specific Antigen PSA for prostate cancer, CA-15.3 for breast cancer and CA-19.9 for pancreatic cancer. However, single tumor markers lack sufficient specificity and sensitivity. Recently, a trend towards small antigen marker sets instead of single markers has emerged, since single markers typically show a lack of specificity. Autoantibody profiling using antigen sets of less than 100 antigens has been applied to identify a variety of cancer types from control sera. The long list of studied cancer types includes glioma [1], intracranial meningioma [2, 3], prostate cancer [4], ovarian cancer [5, 6], lung cancer [7, 8, 9, 10], and head and neck cancer [11, 12]. For the classification of these profiles, web-based tools as the Seroreactivity Pattern Classification Server (SePaCS) [13] have been developed in the recent past.

It has been reported for intracranial meningioma, a mostly benign tumor arising from the meninges covering the brain, that tumor patient' sera and normal sera can be separated with high specificity and sensitivity [Comtesse N, et al. Proc Natl Acad Sci U S A 2005; 102: 9601-9606; Keller A, et al. BMC Bioinformatics 2006; 7:539]. Similar good separation has been achieved for patients with prostate cancer [Wang X, et al. N Engl J Med 2005; 353: 1224-1235]. Long term, depending on the availability of informative antigens, seroreactivity pattern may achieve 100% sensitivity.

In the future, a relatively low-cost technology is necessary in order to provide regular screening of patients' risk groups. In addition, the object is to impose less burden on the patients compared to some imaging technologies like computer tomography (CT).

Therefore, the object of the present invention was to provide a tool to distinguish a tumor disease or cancerous disease from normal and healthy status of an individual. In particular, a general all-embracing cancer screening tool is desirable which allows to generally screen for a tumor disease or cancerous disease in an individual.

The object of the present invention was solved by an in-vitro method or assay for detecting whether or not a human individual has a tumor or a cancerous disease, comprising screening for the presence of antibodies in a blood or serum sample of said human individual, wherein the antibodies are directed to the antigens enolase 1 and latrophilin 1.

In a preferred in-vitro method or assay in addition for the presence of antibodies directed to fascin homolog 1 and vimentin 1 is screened. Further, preferred, in addition for the presence of antibodies directed to replication protein A3 and synuclein is screened. Still further preferred, in addition for the presence of antibodies directed to KIAA1279; calcium calmodulin-dependent protein kinase ID; DEAD (Asp-Glu-Ala-Asp) box polypeptide 54; and inhibitor of growth family, member 4 is screened. Even further preferred, in addition for the presence of antibodies directed to similar to ubiquitin and ribosomal protein S27a precursor; DEAD (Asp-Glu-Ala-Asp) box polypeptide 24; ring finger protein 187; is screened. It is particularly preferred that in addition for the presence of antibodies directed to amyloid beta (A4) precursor protein binding family B, member 1 (FE65); general transcription factor IIB; ribosomal protein L35; glucose phosphate isomerase; centromere protein B, 80KDa; is screened.

A yet another particularly preferred in-vitro method or assay for detecting whether or not a human individual has a tumor or a cancerous disease, comprises the screening for the presence of antibodies in a blood or serum sample of said human individual, wherein the antibodies are directed to the following antigens enolase 1; latrophilin 1; fascin homolog 1; vimentin 1; replication protein A3; synuclein; KIAA1279; calcium calmodulin-dependent protein kinase ID; DEAD (Asp-Glu-Ala-Asp) box polypeptide 54; inhibitor of growth family, member 4; similar to ubiquitin and ribosomal protein S27a precursor; DEAD (Asp-Glu-Ala-Asp) box polypeptide 24; ring finger protein 187; amyloid beta (A4) precursor protein binding family B, member 1 (FE65); general transcription factor IIB; ribosomal protein L35; glucose phosphate isomerase; centromere protein B, 80KDa.

The present invention therefore provides an in-vitro method or assay which makes use of a set of antigens for screening of autoantibodies in the blood serum of an individual in order to evaluate whether or not a human individual has a tumor or a cancerous disease. With this method and assay the present invention provides a tool for a general all-embracing cancer screening. A further advantage of the method or assay according to the present invention is a relatively low-cost technology which may be used for regular screening of patients' risk groups. In addition, the screening method or assay according to the present invention imposes less burden on the patients compared to other imaging technologies or tests.

The method or assay according to the present invention does not make use of a single marker but of at least of 2 or more markers which strongly increases accuracy, sensitivity and specificity of the method or assay compared to single marker tests.

However, any one of the antigens or markers provided according to the present invention is considered to have the potential to provide a reasonable accurate, sensitive and specific marker in a single tumor marker test, screening method or assay for screening of autoantibodies in the blood serum of an individual in order to evaluate whether or not a human individual has a tumor or a cancerous disease.

Therefore, the present invention also provides an in-vitro method or assay for detecting whether or not a human individual has a tumor or a cancerous disease, comprising screening for the presence of antibodies in a blood or serum sample of said human individual, wherein the antibodies are directed to the antigens selected from the group consisting of enolase 1; latrophilin 1; fascin homolog 1; vimentin 1; replication protein A3; synuclein; KIAA1279; calcium calmodulin-dependent protein kinase ID; DEAD (Asp-Glu-Ala-Asp) box polypeptide 54; inhibitor of growth family, member 4; similar to ubiquitin and ribosomal protein S27a precursor; DEAD (Asp-Glu-Ala-Asp) box polypeptide 24; ring finger protein 187; amyloid beta (A4) precursor protein binding family B, member 1 (FE65); general transcription factor IIB; ribosomal protein L35; glucose phosphate isomerase; centromere protein B, 80KDa.

The in-vitro method or assay according to the present invention involves the judgement that the presence of the antibodies directed to one or more of said antigens mentioned before indicate disease state.

The in-vitro method or assay according to the present invention involves the judgement that the presence of the antibodies directed to one or more of said antigens mentioned before indicate the presence of a tumor or a cancerous disease. Preferably, the tumor or cancerous disease is selected from brain cancer, lung cancer and prostate cancer.

The present invention also provides a medicament comprising one or more antibodies directed to an antigen selected from the group consisting of enolase 1; latrophilin 1; fascin homolog 1; vimentin 1; replication protein A3; synuclein; KIAA1279; calcium calmodulin-dependent protein kinase ID; DEAD (Asp-Glu-Ala-Asp) box polypeptide 54; inhibitor of growth family, member 4; similar to ubiquitin and ribosomal protein S27a precursor; DEAD (Asp-Glu-Ala-Asp) box polypeptide 24; ring finger protein 187; amyloid beta (A4) precursor protein binding family B, member 1 (FE65); general transcription factor IIB; ribosomal protein L35; glucose phosphate isomerase; centromere protein B, 80KDa. It is preferred that the medicament is for use in the preventive and/or therapeutic treatment of a tumor or cancerous disease, particularly preferred brain cancer, lung cancer and prostate cancer.

The present invention further provides a medicament comprising one or more antigen(s) selected from the group consisting of enolase 1; latrophilin 1; fascin homolog 1; vimentin 1; replication protein A3; synuclein; KIAA1279; calcium calmodulin-dependent protein kinase ID; DEAD (Asp-Glu-Ala-Asp) box polypeptide 54; inhibitor of growth family, member 4; similar to ubiquitin and ribosomal protein S27a precursor; DEAD (Asp-Glu-Ala-Asp) box polypeptide 24; ring finger protein 187; amyloid beta (A4) precursor protein binding family B, member 1 (FE65); general transcription factor IIB; ribosomal protein L35; glucose phosphate isomerase; centromere protein B, 80KDa. This medicament may be used to immunize an individual by active immunization. It is preferred that the medicament is for use in the preventive and/or therapeutic treatment of a tumor or cancerous disease, particularly preferred brain cancer, lung cancer and prostate cancer

The present invention was completed on the evaluation of specific antigens for its reactivity against serum antibodies in order to assess if an individual has a disease state or a normal healthy state.

In the studies performed to complete the present invention the ability of autoantibody profiling as diagnostic test was confirmed by providing evidence that not only cancer sera can be distinguished well from normal controls, but also from sera of patients with non-cancerous diseases. Altogether, blood sera of 191 cancer patients, 60 physiologically unaffected controls, and 177 sera of patients with non-cancerous diseases were screened for more than 1800 immunogenic clones. The measured autoantibody fingerprints were evaluated using a novel image analysis pipeline.

For 13 antigens, statistically significant (p<0.05) and at least two fold elevated frequency of the immuno-reactivity in cancer sera compared to normal sera could be observed. Nine of these antigens also showed increased reactivity compared to sera of patients with other diseases, including the tumor marker vimentin. Supervised discrimination between cancer and normal sera by using linear Support Vector Machines was possible with an accuracy of 94.04%, a specificity of 83.38%, and a sensitivity of 97.44%. Here, our so-called MIMM (minimally invasive multiple marker) approach showed no significant difference in the classification accuracy between low and higher tumor grades. The classification in healthy and diseased sera showed an even higher accuracy of 96.12% while the discrimination in cancer sera and diseased controls revealed an accuracy of 69.58%.

These results demonstrate that autoantibody profiling offers the possibility of cancer screening for a variety of different cancer types as well as inflammatory diseases at an early disease stage.

The following examples are provided to illustrate the present invention, however, shall not be considered as restricting the invention to the described examples.

### Figures

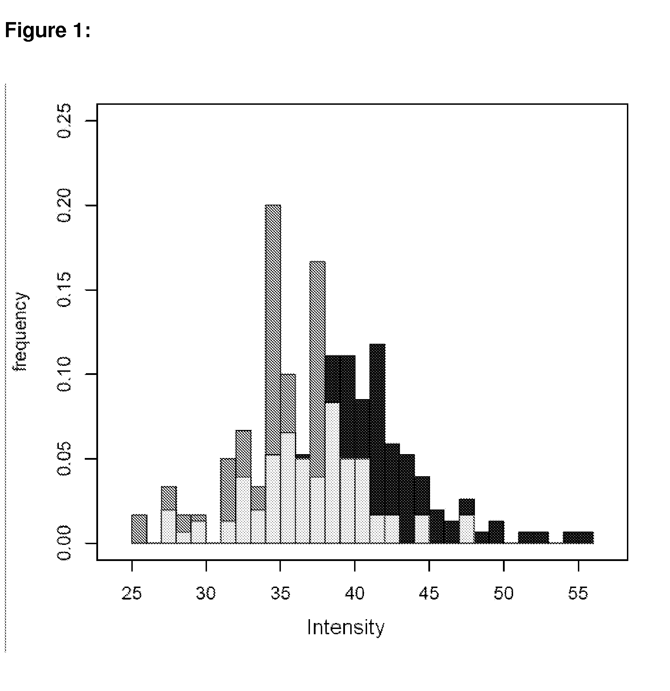

**Figure 1** shows a histogram of intensity values of the marker vimentin. The distribution of cancer sera is indicated in black, the distribution of normal sera is shown in dark grey. The overlap between the distributions is shown in light grey columns.

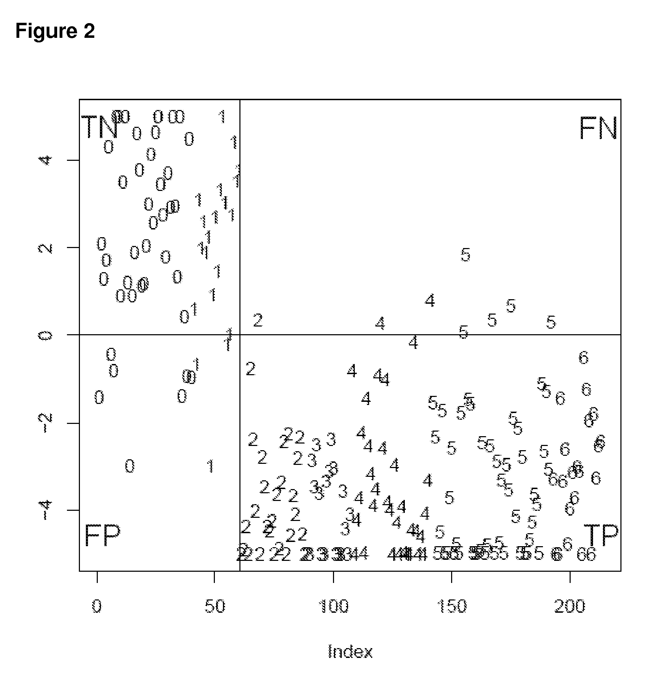

**Figure 2** shows an exemplarily classification result. The y-axis shows the logarithmized quotient of the probability to be in the class "normal" (denoted by 0 and 1) divided by the probability of being in the class "cancer". All sera that are more likely to be in the class "normal" (above the horizontal line) are classified as normal sera. TN denotes true negative predictions, TP true positives, FN false negative predictions, and FP false positives.

### Examples

### General Approach - Overview

Since recent advances occurred in autoantibody profiling for different cancer types, the present inventors carried out studies in respect to whether autoantibodies might offer the option of becoming a general and all-embracing cancer screening method. For this a large-scale autoantibody profiling of 428 blood sera was performed including 191 blood sera of malignant and benign cancer entities of different organs, 60 sera of physiologically unaffected individuals, and 177 sera of patients with various other neurological and inflammatory diseases.

A customized protein array with 1827 immunogenic clones was used to screen for immunoreactivity with the 428 samples. To evaluate the signals, an image analysis procedure was developed and implemented. This procedure computes for each antigen and each sample an intensity value of between 0 and 255. The resulting autoantibody fingerprints were quantile normalized [14] to minimize inter-array effects.

In the following examples the immunity in cancer and control sera are discussed in general. In detail, the reactivity of antigens in the three groups - cancer patients, patients with non-cancer diseases, and normal controls was compared by different measures such as the area under the receiver operator characteristics curve (AUC) or the significance values of Wilcoxon-Mann-Whitney tests [15, 16].

Additionally, it is demonstrated that classification of the relevant fingerprints by linear Support Vector Machines [17] yields highly accurate results. In detail, the three pairwise classifications using Minimally Invasive Multiple Marker (MIMM) approach was carried out: normal control sera vs. cancer sera, normal control sera vs. sera of patients and sera of patients with non-cancer diseases vs. sera of patients with cancer diseases.

The results presented indicate an overall increased humoral immune response in cancer patients and patients with inflammatory diseases. In addition, the study demonstrates that autoantibody profiles offer themselves as a promising approach for non-invasive cancer detection at early stages.

### Example 1: Samples and Screening procedure

Patients' sera: Blood samples of patients and normal controls were obtained from the Departments of neurosurgery and Hemastaseology and Transfusion Medicine of the Saarland University with patient's informed consent. Serum was isolated from the blood serum and subsequently stored as aliquots at -70°C. 428 blood sera were obtained including 191 blood sera of malignant and benign cancer entities of different organs, 60 sera of physiologically unaffected individuals, and 177 sera of patients with various other neurological and inflammatory diseases.

cDNA Expression Library Construction: Poly(A) RNA was isolated from human fetal brain and used to construct the cDNA expression libraries as previously described.

Screening procedure: The applied screening procedure consisted of two steps. (1) A pre-screening to select the clones that show reactivity with pools of patients' sera and (2) a screening of many diseased and control sera using the clones defined in the first step.

Protein macroarray screening: High-density protein arrays consisting of 38,016 *E. coli* expressed proteins from a human fetal brain cDNA expression library were screened. Thereby, a total of 30 serum pools of patients with different cancers (among others meningioma and glioma) and neurodegenerative diseases and inflammatory disease was analyzed. The analysis showed 1827 clones showing reactivity in at least one serum pool. These 1827 disease-related clones were assembled in duplicates on a customized protein array. The resulting arrays were screened with patients and control sera.

Serological spot assays were performed as described previously [Comtesse N et al. Complex humoral immune response against a benign tumor: Frequent antibody response against specific antigens as diagnostic targets Proc Natl Acad Sci USA 2005; 102: 9601-9606]. In brief, nitrocellulose membranes were precoated with a layer of NZCYM/0.7% agarose/2.5mM IPTG and placed on a reservoir layer of NZCYM/0.7% agarose. Monoclonal phage at a concentration of approximately 5,000 pfu/µl were incubated with exponentially growing E. coli XL1 Blue MRF' and spotted on the precoated nitrocellulose membranes. After removal of the agarose film the filters were processed for reactivity with the individual serum samples. The different antigens and the cDNA library as control were spotted in duplicate on the nitrocellulose membrane. For each serum at least two membranes were screened.

Screening was performed with minor variations as described by Horn et al. (Profiling humoral autoimmune repertoire of dilated cardiomyopathy (DCM) patients and development of a disease-associated protein chip. Proteomics. 2006;6(2):605-613.). In brief, macroarrays were washed twice with TBSTT (TBS, 0.05% Tween 20, 0.5% Triton X-1 00) and 4 times with TBS. After blocking with 3% non-fat dry milk powder in TBST (TBS, 0.05% Tween 20), macroarrays were incubated over night with sera 1:1000 diluted in blocking solution (3% non-fat dry milk powder in TBST). After the incubation, sera were stored for the second incubation round. Three washing steps with TBST were followed by incubation with stripping solution at 70°C. Macroarrays were washed twice with TBST and 4 times with TBS. Incubation with blocking solution was followed by the second round of serum incubation over night. Macroarrays were washed three times with TBST, and incubated with secondary antibody (rabbit anti-human IgG, IgA, IgM-Cy5 (H+L)) 1:1000 diluted in blocking solution. Macroarrays were washed four times with TBST, twice with TBS.

After screening, the membranes were scanned using a Typhoon 9410 scanner, with 50 µm resolution, 300 PMT and reacting spots were picked out by a newly developed image analysis procedure. Consequently, a total of 428 blood sera including 191 blood sera of malignant and benign cancer entities of different organs, 60 sera of physiologically unaffected individuals, and 177 sera of patients with various other diseases were investigated. The resultant 428 hybridized filters were processed using a newly developed image analysis pipeline as described below.

### Example 2: Image analysis.

In order to compute intensity values for each protein on each hybridized array an image analysis pipeline was developed. In a pre-processing step, the scanned images were standardized, i.e., slight rotations of the scanned images were corrected and the edges of the image were cut accurately. Thereafter, the array was segmented into regular sub-grids and each protein was assigned a spot target area. By 3-means clustering, each target area was divided into foreground and background pixels. The three clusters corresponded to background pixels, pale foreground pixels and dark foreground pixels. After clustering, if foreground pixels exceeded the originally calculated target area, the previously computed spot areas were enlarged. To this end, a local search for each target area border was carried out separately. In detail, the border was shifted in each direction by up to 5 pixels. For each position, the number of foreground pixels that lay on the border was calculated and the horizontal and vertical border hitting the minimal number of foreground pixels was selected.

For extracting the dark foreground spots from the pale background, the so-called "black top hat" was applied, well known from the field of image analysis [18]. Here, morphological closing is applied, consisting of a dilation step followed by an erosion step. For erosion and dilation, a squared structuring element of size of approximately 40 pixels was applied. Finally, the intensity of each spot was computed as the mean value of all pixels comprising the spot after application of the black top hat operator, i.e. the difference between each of the pixels and the corresponding closed pixel. The completely automatically computed analysis constructed for each protein array an autoantibody reactivity pattern consisting of about 1827 intensity values with a range of between 0 and 255.

Merging replicates: For each antigen, two replicates were spotted on the protein arrays. The respective two values were combined by computing their mean intensity.

Data normalization: To make the arrays directly comparable to each other, a quantile normalization [14] was carried out. Here, it is assumed that most clones are not changed in their reactivity across the arrays. Thus, this normalization may decrease the difference between normal and disease sera.

### Example 3: Statistical Methods

For ranking antigens, several standard methods were applied:

1. AUC: The receiver operator characteristic curve shows sensitivity as function of 1-specificity. If two distributions can be separated completely from each other, the area under the ROC curve (AUC value) computes as 1, if two distributions overlap completely it computes as 0.5. AUC values for each of the antigens were computed to get an idea as to how well this antigen separates serum groups.

2. Wilcoxon-Mann-Whitney testing: For each antigen it was computed whether the distribution in normal and cancer sera differs significantly by carrying out unpaired-two tailed Wilcoxon-Mann-Whitney testing [15, 16]. The significance values of this non-parametric test were adjusted for multiple testing using the Benjamin Hochberg adjustment [19].

3. Reactivity frequencies: Besides the AUC values and t-test significance values, the percentage of reactivity in normal and cancer sera of each antigen is highly important. Thus, the computed intensity values have to be discretized in two bins: reacting and not-reacting. To this end, an intensity threshold of 50 was applied. Antigens with higher intensities were defined as positive, all other antigens as negative. Given this threshold, for each antigen the percentages of cancer and normal sera with intensity values above 50 were computed.

Separation of cancer and normal sera: Separation of cancer and control sera was performed using Support Vector Machines [17] with a linear kernel. Reported accuracy, specificity, sensitivity, and AUC values are averaged values from 100 repetitions of a standard 10-fold cross validation.

The large number of features always suggests the possibility that the applied machine learning technique (in the present case the Support Vector Machine) is adapted too much to the data set. To test for this so-called over-training, stratified permutation tests were carried out. Here, the class labels of the considered classification task were randomly sampled by ensuring that the class probability matches the original class distribution. Again, 100 runs of the 10-fold cross validation were repeated to compute averaged accuracy, specificity and sensitivity.

### Results

A large-scale autoantibody profiling of 428 blood sera including 191 blood sera of malignant and benign cancer entities of different organs, 60 sera of physiologically unaffected individuals, and 177 sera of patients with various other diseases was carried out as described above. For each serum, reactivity values for 1827 immunogenic clones were computed using the newly developed image analysis pipeline. It should be noted that the clones have been spotted in duplicates onto the protein array and the mean value of these replicates was computed. Before analyzing the data, a standard quantile normalization was carried out, to account for differences between the 428 membranes.

### Increased immunoreactivity in cancer sera

For each of the 1827 antigens the area under the receiver operator characteristic (ROC) curve, the percentage of cancer, normal, and other control sera were computed showing reactivity with the antigen. Additionally, p-values were calculated using two-tailed Wilcoxon-Mann-Whitney tests and the p-values for multiple testing were adjusted by controlling the false discovery rate. To compute reactivity frequencies, the data were binarized: each reactivity value higher than 50 was considered as a positive response; all other values as negative.

For 25 clones an at least two-fold increased frequency of the reactivity in cancer sera compared to normal controls and an adjusted p-value of less than 0.05 was detected. Of these 25 clones, 13 could be mapped on to human proteins that were transcribed in the correct reading frame (inframe clones). Table 1 summarizes the antigens occurring with at least two-fold increased frequency in cancer sera compared to normal sera. It was notable that four of these 13 clones reacted more frequently with sera of patients with other diseases compared to cancer sera. The remaining, out of frame clones, represent so-called mimotopes. Details of the inframe clones are provided in Table 2, in which all clones with an at least two fold increased reactivity in cancer sera compared to normal sera were considered.

The highest quotient of reactivity in cancer sera divided by reactivity in normal sera was obtained by enolase 1, showing a 7-fold enrichment of reactivity in cancer sera (p-value of 0.0261). Remarkably, this antigen showed an even higher reactivity in sera of patients with non-cancerous diseases. The best AUC was reached by vimentin 1 that shows a 4-fold enrichment of reactivity (p-value of 0.0001). Moreover, the DEAD box polypeptide 54 reacted in 40% of cancer sera, 15% of normal sera and 21% of other control sera. Likewise, the DEAD box polypeptide 24 reacted in 20% of cancer sera, 8% of normal sera, and 13% of other control sera. The general transcription factor IIB showed reactivity in 29% of cancer sera, 13% of normal sera, and 12% of other sera.

Specifically, no antigens were found that showed an at least 2-fold increased frequency of the reactivity in normal sera compared to cancer sera, confirming the overall increased immunoreactivity in cancer sera.

### Supervised separation of cancer and normal sera

A classification in cancer and control sera was performed using standard linear Support Vector Machines. To compute accuracy rates and confidence intervals of accuracy, specificity and sensitivity, 100 repetitions of the 1 0-fold cross validation was carried out. The reported rates are the average of the 100 repetitions. All classification results are summarized in Table 2.

The classification into cancer sera and normal sera showed an accuracy of 94.08% at a specificity and sensitivity of 83.38% and 97.44%. The permutation tests reached an accuracy of 65.2% at a specificity and sensitivity of 79.57% and 19.47%.

An exemplarily classification result is presented in Figure 2, showing the logarithmized quotient of the probability to be in the class "normal" divided by the probability of being in the class "cancer". Here, all sera with higher probability of being normal, i.e. sera having a logarithmized quotient above zero, are classified as normal. Consequently, 8 cancer sera were not correctly classified, representing so-called false negative (FNs) predictions. These FNs include one non-small cell lung carcinoma, two prostate carcinomas and five gliomas. Additionally, 10 normal sera were predicted to be cancer sera, representing so-called false positive predictions. Remarkably, no small-cell lung carcinoma sera and no meningioma sera were predicted as being normal.

The classification in sera of diseased patients and physiologically normal controls reached an even higher accuracy of 96.12%, given a specificity and sensitivity of 77.48% and 99.16%, respectively. Permutation tests reached an accuracy of 78.44%, specificity of 10.5% and sensitivity of 89.52%.

The classification of cancer and non-cancer sera showed an accuracy, specificity and sensitivity of 69.58%, 67.73%, and 71.29%, while the respective values for the permutation tests were 49.92%, 51.44%, and 48.27%. This comparison demonstrates that the results are better than random, but do not reach the exceptionally high accuracy rates as found in the other two scenarios considered.

### Discussion and Conclusion

The analysis of the reactivity in cancer, non-cancer, and normal sera detected several antigens that are already known to be tumor markers. The most prominent example was vimentin. However, vimentin also showed reactivity in many sera of patients that are diseased but have no cancer. Thus, this protein belongs to the category of disease markers rather than to the category of cancer markers. Moreover, several proteins were detected that have so far not been related to cancer diagnosis. These proteins partially showed decreased reactivity in non-cancer sera compared to cancer sera.

The classification into cancer sera and normal sera as well as the classification into diseased sera and normal sera both showed very high accuracy rates of about 95%. The classification in cancer sera and sera of non-cancer diseases was possible at an accuracy of about 70%, specifying the current frontiers of our molecular disease diagnosis methods.

The accuracy of some of the permutation tests is by far higher than the rate of 50% that one might expect for a two-class scenario. As mentioned in the examples, stratified permutation tests with respect to the class distributions were carried out. In the case of the comparison of normal sera versus diseased sera, 60 to 368 samples were compared. Thus, the permutation tests show a high sensitivity (89.52%) at very low specificity (10.5%). Here, the mean value of specificity and sensitivity (50.01 %) represents a more sensible measure for the actual performance of the permutation tests. The respective mean value for the original class labels computes as 88.32%.

In summary, autoantibody profiling represents an approach that is well-suited for the general detection of various diseases, including cancer. Since the classification results of the present study showed no decreased performance for low-grade tumors, the approach as presented is also suited to the detection of tumors in their early stages, which would, in turn, lead to improved cure rates of the respective tumors.

**Table 1:**

| Antigens occurring with at least two-fold increased frequency in cancer sera compared to normal sera. | | | | | | | | | |
|---|---|---|---|---|---|---|---|---|---|
| **ensembl** | **SEQ ID NO:** | **Gene** | **auc** | **pvalue** | **normal (%)** | **cancer (%)** | **other (%)** | **cancer/ normal** | **other/ normal** |
| ENSG00000074800 | 1 | enolase 1 | 0,64 | 0,0261 | 0,03 | 0,24 | 0,31 | 7,06 | 9,33 |
| ENSG00000072071 | 2 | latrophilin 1 | 0,68 | 0,0051 | 0,05 | 0,22 | 0,24 | 4,31 | 4,89 |
| ENSG00000075618 | 3 | fascin homolog1 | 0,62 | 0,0624 | 0,02 | 0,07 | 0,05 | 3,95 | 3,03 |
| ENSG00000026025 | 4 | vimentin 1 | 0,74 | 0,0001 | 0,12 | 0,46 | 0,39 | 3,92 | 3,38 |
| ENSG00000106399 | 5 | replication protein A3 | 0,59 | 0,1720 | 0,07 | 0,23 | 0,28 | 3,43 | 4,17 |
| ENSG00000064692 | 6 | synuclein | 0,69 | 0,0023 | 0,12 | 0,39 | 0,42 | 3,36 | 3,62 |
| ENSG00000198954 | 7 | KIAA1279 | 0,62 | 0,0721 | 0,07 | 0,20 | 0,18 | 2,94 | 2,67 |
| ENSG00000183049 | 8 | calcium/calmodulin-dependent protein kinase ID | 0,69 | 0,0024 | 0,12 | 0,33 | 0,31 | 2,86 | 2,67 |
| ENSG00000123064 | 9 | DEAD (Asp-Glu-Ala-Asp) box polypeptide 54 | 0,67 | 0,0052 | 0,15 | 0,40 | 0,21 | 2,60 | 1,38 |
| ENSG00000111653 | 10 | inhibitor of growth family, member 4 | 0,67 | 0,0071 | 0,17 | 0,43 | 0,52 | 2,59 | 3,10 |
| ENSG00000198708 | 11 | similar to ubiquitin and ribosomal protein S27a precursor | 0,69 | 0,0024 | 0,22 | 0,52 | 0,52 | 2,41 | 2,41 |
| ENSG00000089737 | 12 | DEAD (Asp-Glu-Ala-Asp) box polypeptide 24 | 0,61 | 0,0826 | 0,08 | 0,20 | 0,13 | 2,35 | 1,60 |
| ENSG00000168159 | 13 | ring finger protein 187 | 0,66 | 0,0097 | 0,15 | 0,35 | 0,30 | 2,31 | 2,03 |
| ENSG00000166313 | 14 | amyloid beta (A4) precursor protein-binding, family B, member 1 (Fe65) | 0,69 | 0,0024 | 0,18 | 0,41 | 0,36 | 2,25 | 1,94 |
| ENSG00000137947 | 15 | general transcription factor IIB | 0,66 | 0,0104 | 0,13 | 0,29 | 0,12 | 2,21 | 0,92 |
| ENSG00000136942 | 16 | ribosomal protein L35 | 0,67 | 0,0051 | 0,25 | 0,54 | 0,51 | 2,14 | 2,04 |
| ENSG00000105220 | 17 | glucose phosphate isomerase | 0,62 | 0,0754 | 0,18 | 0,38 | 0,32 | 2,05 | 1,73 |
| ENSG00000125817 | 18 | centromere protein B, 80kDa | 0,65 | 0,0177 | 0,25 | 0,50 | 0,38 | 2,01 | 1,51 |

**Table 2:**

| Classification outcome for the three classification scenarios. The numbers in squared brackets denote the 95% confidence intervals. | | | |
|---|---|---|---|
| **Classification** | **Accuracy** | **Sensitivity** | **Specificity** |
| Cancer vs. Normal | 94.08% [93.93%-94.22%] | 97.44% [97.32%-97.55%] | 83.38% [82.96%-83.8%] |
| Diseases vs. Normal | 96.12% [96.03%-96.21%] | 99.16% [99.09%-99.22%] | 77.48% [76.99%-77.97%] |
| Cancer vs. Non cancer diseases | 69.58% [69.3%-69.85%] | 71.29% [70.93%-71.66%] | 67.73% [67.33%-68.13%] |

### Bibliography

[1] Ludwig N, Keller A, Comtesse N, Rheinheimer S, Pallasch C, Fischer U, Fassbender K, Steudel WI, Lenhof HP, Meese E. Pattern of serum autoantibodies allows accurate distinction between a tumor and pathologies of the same organ. Clin Cancer Res. 2008 Aug 1;14(15):4767-74.
[2] Comtesse N, Zippel A, Walle S, et al. Complex humoral immune response against a benign tumor: Frequent antibody response against specific antigens as diagnostic targets Proc Natl Acad Sci U S A 2005; 102: 9601-9606.
[3] Keller A, Ludwig N, Comtesse N, Hildebrandt A, Meese E, Lenhof HP. A minimally invasive multiple marker approach allows highly efficient detection of meningioma tumors. BMC Bioinformatics 2006; 7:539.
[4] Wang X, Yu J, Sreekumar A, et al. Autoantibody signatures in prostate cancer. N Engl J Med 2005; 353: 1224-1235.
[5] Chatterjee M, Mohapatra S, lonan A, et al. Diagnostic markers of ovarian cancer by high-throughput antigen cloning and detection on arrays. Cancer Res 2006; 66: 1181-1190.
[6] Erkanli A, Taylor DD, Dean D, et al. Application of Bayesian modeling of autologous antibody responses against ovarian tumor-associated antigens to cancer detection. Cancer Res 2006; 66: 1792-1798.
[7] Chapman CJ, Murray A, McElveen JE, Sahin U, Luxemburger U, Türeci O, Wiewrodt R, Barnes AC, Robertson JF. Autoantibodies in Lung Cancer - possibilities for early detection and subsequent cure. Thorax. 2007 Oct 11
[8] Zhong L, Coe SP, Stromberg AJ, et al. Profiling tumor-associated antibodies for early detection of non-small cell lung cancer. J Thorac Oncol. 2006;1:513-519.
[9] Brichory FM, Misek DE, Yim AM, et al. An immune response manifested by the common occurrence of annexins I and II autoantibodies and high circulating levels of IL-6 in lung cancer. Proc Natl Acad Sci USA. 2001 ;98:9824-9829.
[10] Leidinger P, Keller A, Ludwig N, Rheinheimer S, Hamacher J, Huwer H, Stehle I, Lenhof HP, Meese E. Toward an early diagnosis of lung cancer: an autoantibody signature for squamous cell lung carcinoma. Int J Cancer. 2008 Oct 1;123(7):1631-6.
[11] Vaughan HA, St Clair F, Scanlan MJ, et al. The humoral immune response to head and neck cancer antigens as defined by the serological analysis of tumor antigens by recombinant cDNA expression cloning. Cancer Immun. 2003;4:5.
[12] Heubeck B, Wendler O, Bumm K, Schäfer R, Müller-Vogt U, Häusler M, Meese E, Iro H, Steinhart H. Tumor-associated antigenic pattern in squamous cell carcinomas of the head and neck - Analysed by SEREX. Eur J Cancer. 2006 Jul 10.
[13] Keller A, Comtesse N, Ludwig N, Meese E, Lenhof HP. SePaCS-a web-based application for classification of seroreactivity profiles. Nucleic Acids Res., 35:W683-687, Jul 2007.
[14] Bolstad BM, Irizarry RA, Astrand M, and Speed, TP. A Comparison of Normalization Methods for High Density Oligonucleotide Array Data Based on Bias and Variance. Bioinformatics 2003; 19(2) ,pp 185-193.
[15] Wilcoxon F. Individual comparison by ranking methods. Biometric Bul I, 1:80-83, 1945.
[16] Mann H and Wilcoxon F. On a test of whether one of two random variables is stochastically larger than the other. Ann Mat Stat, 18:50-60, 1947.
[17] Vapnik V. The nature of statistical learning theory, chapter 5.4. Springer, 1995.
[18] Soille H. Morphological image analysis. Springer, 2006.
[19] Benjamini Y, Hochberg, Y. Controlling the false discovery rate: A practical and powerful approach to multiple testing. J R Statist Soc B, 57:289-300, 1995.

## Claims

1. An in-vitro method or assay for detecting whether or not a human individual has a tumor or a cancerous disease, comprising screening for the presence of antibodies in a blood or serum sample of said human individual, wherein the antibodies are directed to the antigens enolase 1 and latrophilin 1.

2. The in-vitro method or assay according to claim 1, wherein in addition for the presence of antibodies directed to fascin homolog 1 and vimentin 1 is screened.

3. The in-vitro method or assay according to claim 1 or 2, wherein in addition for the presence of antibodies directed to replication protein A3 and synuclein is screened.

4. The in-vitro method or assay according to any one of claims 1 to 3, wherein in addition for the presence of antibodies directed to KIAA1279; calcium calmodulin-dependent protein kinase ID; DEAD (Asp-Glu-Ala-Asp) box polypeptide 54; and inhibitor of growth family, member 4 is screened.

5. The in-vitro method or assay according to any one of claims 1 to 4, wherein in addition for the presence of antibodies directed to similar to ubiquitin and ribosomal protein S27a precursor; DEAD (Asp-Glu-Ala-Asp) box polypeptide 24; ring finger protein 187; is screened.

6. The in-vitro method or assay according to any one of claims 1 to 5, wherein in addition for the presence of antibodies directed to amyloid beta (A4) precursor protein binding family B, member 1 (FE65); general transcription factor IIB; ribosomal protein L35; glucose phosphate isomerase; centromere protein B, 80KDa; is screened.

7. An in-vitro method or assay for detecting whether or not a human individual has a tumor or a cancerous disease, comprising screening for the presence of antibodies in a blood or serum sample of said human individual, wherein the antibodies are directed to the following antigens enolase 1; latrophilin 1; fascin homolog 1; vimentin 1; replication protein A3; synuclein; KIAA1279; calcium calmodulin-dependent protein kinase ID; DEAD (Asp-Glu-Ala-Asp) box polypeptide 54; inhibitor of growth family, member 4; similar to ubiquitin and ribosomal protein S27a precursor; DEAD (Asp-Glu-Ala-Asp) box polypeptide 24; ring finger protein 187; amyloid beta (A4) precursor protein binding family B, member 1 (FE65); general transcription factor IIB; ribosomal protein L35; glucose phosphate isomerase; centromere protein B, 80KDa.

8. An in-vitro method or assay for detecting whether or not a human individual has a tumor or a cancerous disease, comprising screening for the presence of antibodies in a blood or serum sample of said human individual, wherein the antibodies are directed to the antigens selected from the group consisting of enolase 1; latrophilin 1; fascin homolog 1; vimentin 1; replication protein A3; synuclein; KIAA1279; calcium calmodulin-dependent protein kinase ID; DEAD (Asp-Glu-Ala-Asp) box polypeptide 54; inhibitor of growth family, member 4; similar to ubiquitin and ribosomal protein S27a precursor; DEAD (Asp-Glu-Ala-Asp) box polypeptide 24; ring finger protein 187; amyloid beta (A4) precursor protein binding family B, member 1 (FE65); general transcription factor IIB; ribosomal protein L35; glucose phosphate isomerase; centromere protein B, 80KDa.

9. The in-vitro method or assay according to any one of claims 1 to 8, wherein the presence of the antibodies directed to said antigens indicate disease state.

10. The in-vitro method or assay according to any one of claims 1 to 9, wherein the presence of the antibodies directed to said antigens indicate the presence of a tumor or a cancerous disease.

11. The in-vitro method or assay according to claim 10, wherein the tumor or cancerous disease is selected from brain cancer, lung cancer and prostate cancer.

12. A medicament comprising one or more antibodies directed to an antigen selected from the group consisting of enolase 1; latrophilin 1; fascin homolog 1; vimentin 1; replication protein A3; synuclein; KIAA1279; calcium calmodulin-dependent protein kinase ID; DEAD (Asp-Glu-Ala-Asp) box polypeptide 54; inhibitor of growth family, member 4; similar to ubiquitin and ribosomal protein S27a precursor; DEAD (Asp-Glu-Ala-Asp) box polypeptide 24; ring finger protein 187; amyloid beta (A4) precursor protein binding family B, member 1 (FE65); general transcription factor IIB; ribosomal protein L35; glucose phosphate isomerase; centromere protein B, 80KDa.

13. A medicament comprising one or more antigen(s) selected from the group consisting of enolase 1; latrophilin 1; fascin homolog 1; vimentin 1; replication protein A3; synuclein; KIAA1279; calcium calmodulin-dependent protein kinase ID; DEAD (Asp-Glu-Ala-Asp) box polypeptide 54; inhibitor of growth family, member 4; similar to ubiquitin and ribosomal protein S27a precursor; DEAD (Asp-Glu-Ala-Asp) box polypeptide 24; ring finger protein 187; amyloid beta (A4) precursor protein binding family B, member 1 (FE65); general transcription factor IIB; ribosomal protein L35; glucose phosphate isomerase; centromere protein B, 80KDa.

14. The medicament according to claim 12 or 13, for use in the preventive and/or therapeutic treatment of a tumor or cancerous disease.

15. The medicament according to claim 14, wherein the tumor or cancerous disease is selected from brain cancer, lung cancer and prostate cancer.


SEQUENCE LISTING

<110>  Universität des Saarlandes

<120>  An in-vitro method or assay for detecting a tumor or a
cancerous
       disease by screening for antibodies

<130>  P22221EP

<160>  18

<170>  PatentIn version 3.3

<210>  1
<211>  434
<212>  PRT
<213>  Homo sapiens

<400>  1

Met Ser Ile Leu Lys Ile His Ala Arg Glu Ile Phe Asp Ser Arg Gly
1               5                   10                  15

Asn Pro Thr Val Glu Val Asp Leu Phe Thr Ser Lys Gly Leu Phe Arg
            20                  25                  30

Ala Ala Val Pro Ser Gly Ala Ser Thr Gly Ile Tyr Glu Ala Leu Glu
        35                  40                  45

Leu Arg Asp Asn Asp Lys Thr Arg Tyr Met Gly Lys Gly Val Ser Lys
    50                  55                  60

Ala Val Glu His Ile Asn Lys Thr Ile Ala Pro Ala Leu Val Ser Lys
65                  70                  75                  80

Lys Leu Asn Val Thr Glu Gln Glu Lys Ile Asp Lys Leu Met Ile Glu
                85                  90                  95

Met Asp Gly Thr Glu Asn Lys Ser Lys Phe Gly Ala Asn Ala Ile Leu
            100                 105                 110

Gly Val Ser Leu Ala Val Cys Lys Ala Gly Ala Val Glu Lys Gly Val
        115                 120                 125

Pro Leu Tyr Arg His Ile Ala Asp Leu Ala Gly Asn Ser Glu Val Ile
    130                 135                 140

Leu Pro Val Pro Ala Phe Asn Val Ile Asn Gly Gly Ser His Ala Gly
145                 150                 155                 160

Asn Lys Leu Ala Met Gln Glu Phe Met Ile Leu Pro Val Gly Ala Ala
                165                 170                 175

Asn Phe Arg Glu Ala Met Arg Ile Gly Ala Glu Val Tyr His Asn Leu
            180                 185                 190

Lys Asn Val Ile Lys Glu Lys Tyr Gly Lys Asp Ala Thr Asn Val Gly
        195                 200                 205

Asp Glu Gly Gly Phe Ala Pro Asn Ile Leu Glu Asn Lys Glu Gly Leu
    210                 215                 220

Glu Leu Leu Lys Thr Ala Ile Gly Lys Ala Gly Tyr Thr Asp Lys Val
225                 230                 235                 240

Val Ile Gly Met Asp Val Ala Ala Ser Glu Phe Phe Arg Ser Gly Lys
                245                 250                 255

Tyr Asp Leu Asp Phe Lys Ser Pro Asp Asp Pro Ser Arg Tyr Ile Ser
            260                 265                 270

Pro Asp Gln Leu Ala Asp Leu Tyr Lys Ser Phe Ile Lys Asp Tyr Pro
        275                 280                 285

Val Val Ser Ile Glu Asp Pro Phe Asp Gln Asp Asp Trp Gly Ala Trp
    290                 295                 300

Gln Lys Phe Thr Ala Ser Ala Gly Ile Gln Val Val Gly Asp Asp Leu
305                 310                 315                 320

Thr Val Thr Asn Pro Lys Arg Ile Ala Lys Ala Val Asn Glu Lys Ser
                325                 330                 335

Cys Asn Cys Leu Leu Leu Lys Val Asn Gln Ile Gly Ser Val Thr Glu

340                 345                 350

Ser Leu Gln Ala Cys Lys Leu Ala Gln Ala Asn Gly Trp Gly Val Met
        355                 360                 365

Val Ser His Arg Ser Gly Glu Thr Glu Asp Thr Phe Ile Ala Asp Leu
    370                 375                 380

Val Val Gly Leu Cys Thr Gly Gln Ile Lys Thr Gly Ala Pro Cys Arg
385                 390                 395                 400

Ser Glu Arg Leu Ala Lys Tyr Asn Gln Leu Leu Arg Ile Glu Glu Glu
                405                 410                 415

Leu Gly Ser Lys Ala Lys Phe Ala Gly Arg Asn Phe Arg Asn Pro Leu
            420                 425                 430

Ala Lys

<210>  2
<211>  1474
<212>  PRT
<213>  Homo sapiens

<400>  2

<210>  3
<211>  493
<212>  PRT
<213>  Homo sapiens

<400>  3

Met Thr Ala Asn Gly Thr Ala Glu Ala Val Gln Ile Gln Phe Gly Leu
1               5                   10                  15

Ile Asn Cys Gly Asn Lys Tyr Leu Thr Ala Glu Ala Phe Gly Phe Lys
            20                  25                  30

Val Asn Ala Ser Ala Ser Ser Leu Lys Lys Lys Gln Ile Trp Thr Leu
        35                  40                  45

Glu Gln Pro Pro Asp Glu Ala Gly Ser Ala Ala Val Cys Leu Arg Ser
    50                  55                  60

His Leu Gly Arg Tyr Leu Ala Ala Asp Lys Asp Gly Asn Val Thr Cys
65                  70                  75                  80

Glu Arg Glu Val Pro Gly Pro Asp Cys Arg Phe Leu Ile Val Ala His
                85                  90                  95

Asp Asp Gly Arg Trp Ser Leu Gln Ser Glu Ala His Arg Arg Tyr Phe
            100                 105                 110

Gly Gly Thr Glu Asp Arg Leu Ser Cys Phe Ala Gln Thr Val Ser Pro
        115                 120                 125

Ala Glu Lys Trp Ser Val His Ile Ala Met His Pro Gln Val Asn Ile
    130                 135                 140

Tyr Ser Val Thr Arg Lys Arg Tyr Ala His Leu Ser Ala Arg Pro Ala
145                 150                 155                 160

Asp Glu Ile Ala Val Asp Arg Asp Val Pro Trp Gly Val Asp Ser Leu
                165                 170                 175

Ile Thr Leu Ala Phe Gln Asp Gln Arg Tyr Ser Val Gln Thr Ala Asp
            180                 185                 190

His Arg Phe Leu Arg His Asp Gly Arg Leu Val Ala Arg Pro Glu Pro
        195                 200                 205

Ala Thr Gly Tyr Thr Leu Glu Phe Arg Ser Gly Lys Val Ala Phe Arg
    210                 215                 220

Asp Cys Glu Gly Arg Tyr Leu Ala Pro Ser Gly Pro Ser Gly Thr Leu
225                 230                 235                 240

Lys Ala Gly Lys Ala Thr Lys Val Gly Lys Asp Glu Leu Phe Ala Leu
                245                 250                 255

Glu Gln Ser Cys Ala Gln Val Val Leu Gln Ala Ala Asn Glu Arg Asn
            260                 265                 270

Val Ser Thr Arg Gln Gly Met Asp Leu Ser Ala Asn Gln Asp Glu Glu
        275                 280                 285

Thr Asp Gln Glu Thr Phe Gln Leu Glu Ile Asp Arg Asp Thr Lys Lys
    290                 295                 300

Cys Ala Phe Arg Thr His Thr Gly Lys Tyr Trp Thr Leu Thr Ala Thr
305                 310                 315                 320

Gly Gly Val Gln Ser Thr Ala Ser Ser Lys Asn Ala Ser Cys Tyr Phe
                325                 330                 335

Asp Ile Glu Trp Arg Asp Arg Arg Ile Thr Leu Arg Ala Ser Asn Gly
            340                 345                 350

Lys Phe Val Thr Ser Lys Lys Asn Gly Gln Leu Ala Ala Ser Val Glu
        355                 360                 365

Thr Ala Gly Asp Ser Glu Leu Phe Leu Met Lys Leu Ile Asn Arg Pro
    370                 375                 380

Ile Ile Val Phe Arg Gly Glu His Gly Phe Ile Gly Cys Arg Lys Val
385                 390                 395                 400

Thr Gly Thr Leu Asp Ala Asn Arg Ser Ser Tyr Asp Val Phe Gln Leu
                405                 410                 415

Glu Phe Asn Asp Gly Ala Tyr Asn Ile Lys Asp Ser Thr Gly Lys Tyr
            420                 425                 430

Trp Thr Val Gly Ser Asp Ser Ala Val Thr Ser Ser Gly Asp Thr Pro

435                 440                 445

Val Asp Phe Phe Phe Glu Phe Cys Asp Tyr Asn Lys Val Ala Ile Lys
    450                 455                 460

Val Gly Gly Arg Tyr Leu Lys Gly Asp His Ala Gly Val Leu Lys Ala
465                 470                 475                 480

Ser Ala Glu Thr Val Asp Pro Ala Ser Leu Trp Glu Tyr
                485                 490

<210>  4
<211>  466
<212>  PRT
<213>  Homo sapiens

<400>  4

Met Ser Thr Arg Ser Val Ser Ser Ser Ser Tyr Arg Arg Met Phe Gly
1               5                   10                  15

Gly Pro Gly Thr Ala Ser Arg Pro Ser Ser Ser Arg Ser Tyr Val Thr
            20                  25                  30

Thr Ser Thr Arg Thr Tyr Ser Leu Gly Ser Ala Leu Arg Pro Ser Thr
        35                  40                  45

Ser Arg Ser Leu Tyr Ala Ser Ser Pro Gly Gly Val Tyr Ala Thr Arg
    50                  55                  60

Ser Ser Ala Val Arg Leu Arg Ser Ser Val Pro Gly Val Arg Leu Leu
65                  70                  75                  80

Gln Asp Ser Val Asp Phe Ser Leu Ala Asp Ala Ile Asn Thr Glu Phe
                85                  90                  95

Lys Asn Thr Arg Thr Asn Glu Lys Val Glu Leu Gln Glu Leu Asn Asp
            100                 105                 110

Arg Phe Ala Asn Tyr Ile Asp Lys Val Arg Phe Leu Glu Gln Gln Asn
        115                 120                 125

Lys Ile Leu Leu Ala Glu Leu Glu Gln Leu Lys Gly Gln Gly Lys Ser
    130                 135                 140

Arg Leu Gly Asp Leu Tyr Glu Glu Glu Met Arg Glu Leu Arg Arg Gln
145                 150                 155                 160

Val Asp Gln Leu Thr Asn Asp Lys Ala Arg Val Glu Val Glu Arg Asp
                165                 170                 175

Asn Leu Ala Glu Asp Ile Met Arg Leu Arg Glu Lys Leu Gln Glu Glu
            180                 185                 190

Met Leu Gln Arg Glu Glu Ala Glu Asn Thr Leu Gln Ser Phe Arg Gln
        195                 200                 205

Asp Val Asp Asn Ala Ser Leu Ala Arg Leu Asp Leu Glu Arg Lys Val
    210                 215                 220

Glu Ser Leu Gln Glu Glu Ile Ala Phe Leu Lys Lys Leu His Glu Glu
225                 230                 235                 240

Glu Ile Gln Glu Leu Gln Ala Gln Ile Gln Glu Gln His Val Gln Ile
                245                 250                 255

Asp Val Asp Val Ser Lys Pro Asp Leu Thr Ala Ala Leu Arg Asp Val
            260                 265                 270

Arg Gln Gln Tyr Glu Ser Val Ala Ala Lys Asn Leu Gln Glu Ala Glu
        275                 280                 285

Glu Trp Tyr Lys Ser Lys Phe Ala Asp Leu Ser Glu Ala Ala Asn Arg
    290                 295                 300

Asn Asn Asp Ala Leu Arg Gln Ala Lys Gln Glu Ser Thr Glu Tyr Arg
305                 310                 315                 320

Arg Gln Val Gln Ser Leu Thr Cys Glu Val Asp Ala Leu Lys Gly Thr
                325                 330                 335

Asn Glu Ser Leu Glu Arg Gln Met Arg Glu Met Glu Glu Asn Phe Ala
            340                 345                 350

Val Glu Ala Ala Asn Tyr Gln Asp Thr Ile Gly Arg Leu Gln Asp Glu
        355                 360                 365

Ile Gln Asn Met Lys Glu Glu Met Ala Arg His Leu Arg Glu Tyr Gln
    370                 375                 380

Asp Leu Leu Asn Val Lys Met Ala Leu Asp Ile Glu Ile Ala Thr Tyr
385                 390                 395                 400

Arg Lys Leu Leu Glu Gly Glu Glu Ser Arg Ile Ser Leu Pro Leu Pro
                405                 410                 415

Asn Phe Ser Ser Leu Asn Leu Arg Glu Thr Asn Leu Asp Ser Leu Pro
            420                 425                 430

Leu Val Asp Thr His Ser Lys Arg Thr Leu Leu Ile Lys Thr Val Glu
        435                 440                 445

Thr Arg Asp Gly Gln Val Ile Asn Glu Thr Ser Gln His His Asp Asp
    450                 455                 460

Leu Glu
465

<210>  5
<211>  121
<212>  PRT
<213>  Homo sapiens

<400>  5

Met Val Asp Met Met Asp Leu Pro Arg Ser Arg Ile Asn Ala Gly Met
1               5                   10                  15

Leu Ala Gln Phe Ile Asp Lys Pro Val Cys Phe Val Gly Arg Leu Glu
            20                  25                  30

Lys Ile His Pro Thr Gly Lys Met Phe Ile Leu Ser Asp Gly Glu Gly
        35                  40                  45

Lys Asn Gly Thr Ile Glu Leu Met Glu Pro Leu Asp Glu Glu Ile Ser
    50                  55                  60

Gly Ile Val Glu Val Val Gly Arg Val Thr Ala Lys Ala Thr Ile Leu
65                  70                  75                  80

Cys Thr Ser Tyr Val Gln Phe Lys Glu Asp Ser His Pro Phe Asp Leu
                85                  90                  95

Gly Leu Tyr Asn Glu Ala Val Lys Ile Ile His Asp Phe Pro Gln Phe
            100                 105                 110

Tyr Pro Leu Gly Ile Val Gln His Asp
        115                 120

<210>  6
<211>  919
<212>  PRT
<213>  Homo sapiens

<400>  6

Met Glu Ala Pro Glu Tyr Leu Asp Leu Asp Glu Ile Asp Phe Ser Asp
1               5                   10                  15

Asp Ile Ser Tyr Ser Val Thr Ser Leu Lys Thr Ile Pro Glu Leu Cys
            20                  25                  30

Arg Arg Cys Asp Thr Gln Asn Glu Asp Arg Ser Val Ser Ser Ser Ser
        35                  40                  45

Trp Asn Cys Gly Ile Ser Thr Leu Ile Thr Asn Thr Gln Lys Pro Thr
    50                  55                  60

Gly Ile Ala Asp Val Tyr Ser Lys Phe Arg Pro Val Lys Arg Val Ser
65                  70                  75                  80

Pro Leu Lys His Gln Pro Glu Thr Leu Glu Asn Asn Glu Ser Asp Asp
                85                  90                  95

Gln Lys Asn Gln Lys Val Val Glu Tyr Gln Lys Gly Gly Glu Ser Asp
            100                 105                 110

Leu Gly Pro Gln Pro Gln Glu Leu Gly Pro Gly Asp Gly Val Gly Gly
        115                 120                 125

Pro Pro Gly Lys Ser Ser Glu Pro Ser Thr Ser Leu Gly Glu Leu Glu
    130                 135                 140

His Tyr Asp Leu Asp Met Asp Glu Ile Leu Asp Val Pro Tyr Ile Lys
145                 150                 155                 160

Ser Ser Gln Gln Leu Ala Ser Phe Thr Lys Val Thr Ser Glu Lys Arg
                165                 170                 175

Ile Leu Gly Leu Cys Thr Thr Ile Asn Gly Leu Ser Gly Lys Ala Cys
            180                 185                 190

Ser Thr Gly Ser Ser Glu Ser Ser Ser Ser Asn Met Ala Pro Phe Cys
        195                 200                 205

Val Leu Ser Pro Val Lys Ser Pro His Leu Arg Lys Ala Ser Ala Val
    210                 215                 220

Ile His Asp Gln His Lys Leu Ser Thr Glu Glu Thr Glu Ile Ser Pro
225                 230                 235                 240

Pro Leu Val Lys Cys Gly Ser Ala Tyr Glu Pro Glu Asn Gln Ser Lys
                245                 250                 255

Asp Phe Leu Asn Lys Thr Phe Ser Asp Pro His Gly Arg Lys Val Glu
            260                 265                 270

Lys Thr Thr Pro Asp Cys Gln Leu Arg Ala Phe His Leu Gln Ser Ser
        275                 280                 285

Ala Ala Glu Ser Lys Pro Glu Glu Gln Val Ser Gly Leu Asn Arg Thr
    290                 295                 300

Ser Ser Gln Gly Pro Glu Glu Arg Ser Glu Tyr Leu Lys Lys Val Lys

305                 310                 315                 320

Ser Ile Leu Asn Ile Val Lys Glu Gly Gln Ile Ser Leu Leu Pro His
                325                 330                 335

Leu Ala Ala Asp Asn Leu Asp Lys Ile His Asp Glu Asn Gly Asn Asn
            340                 345                 350

Leu Leu His Ile Ala Ala Ser Gln Gly His Ala Glu Cys Leu Gln His
        355                 360                 365

Leu Thr Ser Leu Met Gly Glu Asp Cys Leu Asn Glu Arg Asn Thr Glu
    370                 375                 380

Lys Leu Thr Pro Ala Gly Leu Ala Ile Lys Asn Gly Gln Leu Glu Cys
385                 390                 395                 400

Val Arg Trp Met Val Ser Glu Thr Glu Ala Ile Ala Glu Leu Ser Cys
                405                 410                 415

Ser Lys Asp Phe Pro Ser Leu Ile His Tyr Ala Gly Cys Tyr Gly Gln
            420                 425                 430

Glu Lys Ile Leu Leu Trp Leu Leu Gln Phe Met Gln Glu Gln Gly Ile
        435                 440                 445

Ser Leu Asp Glu Val Asp Gln Asp Gly Asn Ser Ala Val His Val Ala
    450                 455                 460

Ser Gln His Gly Tyr Leu Gly Cys Ile Gln Thr Leu Val Glu Tyr Gly
465                 470                 475                 480

Ala Asn Val Thr Met Gln Asn His Ala Gly Glu Lys Pro Ser Gln Ser
                485                 490                 495

Ala Glu Arg Gln Gly His Thr Leu Cys Ser Arg Tyr Leu Val Val Val
            500                 505                 510

Glu Thr Cys Met Ser Leu Ala Ser Gln Val Val Lys Leu Thr Lys Gln
        515                 520                 525

Leu Lys Glu Gln Thr Val Glu Arg Val Thr Leu Gln Asn Gln Leu Gln
    530                 535                 540

Gln Phe Leu Glu Ala Gln Lys Ser Glu Gly Lys Ser Leu Pro Ser Ser
545                 550                 555                 560

Pro Ser Ser Pro Ser Ser Pro Ala Ser Arg Lys Ser Gln Trp Lys Ser
                565                 570                 575

Pro Asp Ala Asp Asp Asp Ser Val Ala Lys Ser Lys Pro Gly Val Gln
            580                 585                 590

Glu Gly Ile Gln Val Leu Gly Ser Leu Ser Ala Ser Ser Arg Ala Arg
        595                 600                 605

Pro Lys Ala Lys Asp Glu Asp Ser Asp Lys Ile Leu Arg Gln Leu Leu
    610                 615                 620

Gly Lys Glu Ile Ser Glu Asn Val Cys Thr Gln Glu Lys Leu Ser Leu
625                 630                 635                 640

Glu Phe Gln Asp Ala Gln Ala Ser Ser Arg Asn Ser Lys Lys Ile Pro
                645                 650                 655

Leu Glu Lys Arg Glu Leu Lys Leu Ala Arg Leu Arg Gln Leu Met Gln
            660                 665                 670

Arg Ser Leu Ser Glu Ser Asp Thr Asp Ser Asn Asn Ser Glu Asp Pro
        675                 680                 685

Lys Thr Thr Pro Val Arg Lys Ala Asp Arg Pro Arg Pro Gln Pro Ile
    690                 695                 700

Val Glu Ser Val Glu Ser Met Asp Ser Ala Glu Ser Leu His Leu Met
705                 710                 715                 720

Ile Lys Lys His Thr Leu Ala Ser Gly Gly Arg Arg Phe Pro Phe Ser
                725                 730                 735

Ile Lys Ala Ser Lys Ser Leu Asp Gly His Ser Pro Ser Pro Thr Ser
            740                 745                 750

Glu Ser Ser Glu Pro Asp Leu Glu Ser Gln Tyr Pro Gly Ser Gly Ser
        755                 760                 765

Ile Pro Pro Asn Gln Pro Ser Gly Asp Pro Gln Gln Pro Ser Pro Asp
    770                 775                 780

Ser Thr Ala Ala Gln Lys Val Ala Thr Ser Pro Lys Ser Ala Leu Lys
785                 790                 795                 800

Ser Pro Ser Ser Lys Arg Arg Thr Ser Gln Asn Leu Lys Leu Arg Val
                805                 810                 815

Thr Phe Glu Glu Pro Val Val Gln Met Glu Gln Pro Ser Leu Glu Leu
            820                 825                 830

Asn Gly Glu Lys Asp Lys Asp Lys Gly Arg Thr Leu Gln Arg Thr Ser
        835                 840                 845

Thr Ser Asn Glu Ser Gly Asp Gln Leu Lys Arg Pro Phe Gly Ala Phe
    850                 855                 860

Arg Ser Ile Met Glu Thr Leu Ser Gly Asn Gln Asn Asn Asn Asn Asn
865                 870                 875                 880

Tyr Gln Ala Ala Asn Gln Leu Lys Thr Ser Thr Leu Pro Leu Thr Ser
                885                 890                 895

Leu Gly Arg Lys Thr Asp Ala Lys Gly Asn Pro Ala Ser Ser Ala Ser
            900                 905                 910

Lys Gly Lys Asn Lys Ala Ala
        915

<210>  7
<211>  621
<212>  PRT
<213>  Homo sapiens

<400>  7

Met Ala Asn Val Pro Trp Ala Glu Val Cys Glu Lys Phe Gln Ala Ala
1               5                   10                  15

Leu Ala Leu Ser Arg Val Glu Leu His Lys Asn Pro Glu Lys Glu Pro
            20                  25                  30

Tyr Lys Ser Lys Tyr Ser Ala Arg Ala Leu Leu Glu Glu Val Lys Ala
        35                  40                  45

Leu Leu Gly Pro Ala Pro Glu Asp Glu Asp Glu Arg Pro Glu Ala Glu
    50                  55                  60

Asp Gly Pro Gly Ala Gly Asp His Ala Leu Gly Leu Pro Ala Glu Val
65                  70                  75                  80

Val Glu Pro Glu Gly Pro Val Ala Gln Arg Ala Val Arg Leu Ala Val
                85                  90                  95

Ile Glu Phe His Leu Gly Val Asn His Ile Asp Thr Glu Glu Leu Ser
            100                 105                 110

Ala Gly Glu Glu His Leu Val Lys Cys Leu Arg Leu Leu Arg Arg Tyr
        115                 120                 125

Arg Leu Ser His Asp Cys Ile Ser Leu Cys Ile Gln Ala Gln Asn Asn
    130                 135                 140

Leu Gly Ile Leu Trp Ser Glu Arg Glu Glu Ile Glu Thr Ala Gln Ala
145                 150                 155                 160

Tyr Leu Glu Ser Ser Glu Ala Leu Tyr Asn Gln Tyr Met Lys Glu Val
                165                 170                 175

Gly Ser Pro Pro Leu Asp Pro Thr Glu Arg Phe Leu Pro Glu Glu Glu
            180                 185                 190

Lys Leu Thr Glu Gln Glu Arg Ser Lys Arg Phe Glu Lys Val Tyr Thr
        195                 200                 205

His Asn Leu Tyr Tyr Leu Ala Gln Val Tyr Gln His Leu Glu Met Phe
    210                 215                 220

Glu Lys Ala Ala His Tyr Cys His Ser Thr Leu Lys Arg Gln Leu Glu
225                 230                 235                 240

His Asn Ala Tyr His Pro Ile Glu Trp Ala Ile Asn Ala Ala Thr Leu
                245                 250                 255

Ser Gln Phe Tyr Ile Asn Lys Leu Cys Phe Met Glu Ala Arg His Cys
            260                 265                 270

Leu Ser Ala Ala Asn Val Ile Phe Gly Gln Thr Gly Lys Ile Ser Ala
        275                 280                 285

Thr Glu Asp Thr Pro Glu Ala Glu Gly Glu Val Pro Glu Leu Tyr His
    290                 295                 300

Gln Arg Lys Gly Glu Ile Ala Arg Cys Trp Ile Lys Tyr Cys Leu Thr
305                 310                 315                 320

Leu Met Gln Asn Ala Gln Leu Ser Met Gln Asp Asn Ile Gly Glu Leu
                325                 330                 335

Asp Leu Asp Lys Gln Ser Glu Leu Arg Ala Leu Arg Lys Lys Glu Leu
            340                 345                 350

Asp Glu Glu Glu Ser Ile Arg Lys Lys Ala Val Gln Phe Gly Thr Gly
        355                 360                 365

Glu Leu Cys Asp Ala Ile Ser Ala Val Glu Glu Lys Val Ser Tyr Leu
    370                 375                 380

Arg Pro Leu Asp Phe Glu Glu Ala Arg Glu Leu Phe Leu Leu Gly Gln
385                 390                 395                 400

His Tyr Val Phe Glu Ala Lys Glu Phe Phe Gln Ile Asp Gly Tyr Val
                405                 410                 415

Thr Asp His Ile Glu Val Val Gln Asp His Ser Ala Leu Phe Lys Val
            420                 425                 430

Leu Ala Phe Phe Glu Thr Asp Met Glu Arg Arg Cys Lys Met His Lys
        435                 440                 445

Arg Arg Ile Ala Met Leu Glu Pro Leu Thr Val Asp Leu Asn Pro Gln
    450                 455                 460

Tyr Tyr Leu Leu Val Asn Arg Gln Ile Gln Phe Glu Ile Ala His Ala
465                 470                 475                 480

Tyr Tyr Asp Met Met Asp Leu Lys Val Ala Ile Ala Asp Arg Leu Arg
                485                 490                 495

Asp Pro Asp Ser His Ile Val Lys Lys Ile Asn Asn Leu Asn Lys Ser
            500                 505                 510

Ala Leu Lys Tyr Tyr Gln Leu Phe Leu Asp Ser Leu Arg Asp Pro Asn
        515                 520                 525

Lys Val Phe Pro Glu His Ile Gly Glu Asp Val Leu Arg Pro Ala Met
    530                 535                 540

Leu Ala Lys Phe Arg Val Ala Arg Leu Tyr Gly Lys Ile Ile Thr Ala
545                 550                 555                 560

Asp Pro Lys Lys Glu Leu Glu Asn Leu Ala Thr Ser Leu Glu His Tyr
                565                 570                 575

Lys Phe Ile Val Asp Tyr Cys Glu Lys His Pro Glu Ala Ala Gln Glu
            580                 585                 590

Ile Glu Val Glu Leu Glu Leu Ser Lys Glu Met Val Ser Leu Leu Pro
        595                 600                 605

Thr Lys Met Glu Arg Phe Arg Thr Lys Met Ala Leu Thr
    610                 615                 620

<210>  8
<211>  357
<212>  PRT
<213>  Homo sapiens

<400>  8

Met Ala Arg Glu Asn Gly Glu Ser Ser Ser Ser Trp Lys Lys Gln Ala
1               5                   10                  15

Glu Asp Ile Lys Lys Ile Phe Glu Phe Lys Glu Thr Leu Gly Thr Gly
            20                  25                  30

Ala Phe Ser Glu Val Val Leu Ala Glu Glu Lys Ala Thr Gly Lys Leu
        35                  40                  45

Phe Ala Val Lys Cys Ile Pro Lys Lys Ala Leu Lys Gly Lys Glu Ser
    50                  55                  60

Ser Ile Glu Asn Glu Ile Ala Val Leu Arg Lys Ile Lys His Glu Asn
65                  70                  75                  80

Ile Val Ala Leu Glu Asp Ile Tyr Glu Ser Pro Asn His Leu Tyr Leu
                85                  90                  95

Val Met Gln Leu Val Ser Gly Gly Glu Leu Phe Asp Arg Ile Val Glu
            100                 105                 110

Lys Gly Phe Tyr Thr Glu Lys Asp Ala Ser Thr Leu Ile Arg Gln Val
        115                 120                 125

Leu Asp Ala Val Tyr Tyr Leu His Arg Met Gly Ile Val His Arg Asp
    130                 135                 140

Leu Lys Pro Glu Asn Leu Leu Tyr Tyr Ser Gln Asp Glu Glu Ser Lys
145                 150                 155                 160

Ile Met Ile Ser Asp Phe Gly Leu Ser Lys Met Glu Gly Lys Gly Asp
                165                 170                 175

Val Met Ser Thr Ala Cys Gly Thr Pro Gly Tyr Val Ala Pro Glu Val
            180                 185                 190

Leu Ala Gln Lys Pro Tyr Ser Lys Ala Val Asp Cys Trp Ser Ile Gly
        195                 200                 205

Val Ile Ala Tyr Ile Leu Leu Cys Gly Tyr Pro Pro Phe Tyr Asp Glu
    210                 215                 220

Asn Asp Ser Lys Leu Phe Glu Gln Ile Leu Lys Ala Glu Tyr Glu Phe
225                 230                 235                 240

Asp Ser Pro Tyr Trp Asp Asp Ile Ser Asp Ser Ala Lys Asp Phe Ile
                245                 250                 255

Arg Asn Leu Met Glu Lys Asp Pro Asn Lys Arg Tyr Thr Cys Glu Gln
            260                 265                 270

Ala Ala Arg His Pro Trp Ile Ala Gly Asp Thr Ala Leu Asn Lys Asn
        275                 280                 285

Ile His Glu Ser Val Ser Ala Gln Ile Arg Lys Asn Phe Ala Lys Ser
    290                 295                 300

Lys Trp Arg Gln Ala Phe Asn Ala Thr Ala Val Val Arg His Met Arg
305                 310                 315                 320

Lys Leu His Leu Gly Ser Ser Leu Asp Ser Ser Asn Ala Ser Val Ser
                325                 330                 335

Ser Ser Leu Ser Leu Ala Ser Gln Lys Asp Cys Ala Tyr Val Ala Lys
            340                 345                 350

Pro Glu Ser Leu Ser
        355

<210>  9
<211>  882
<212>  PRT
<213>  Homo sapiens

<400>  9

Met Ala Ala Asp Lys Gly Pro Ala Ala Gly Pro Arg Ser Arg Ala Ala
1               5                   10                  15

Met Ala Gln Trp Arg Lys Lys Lys Gly Leu Arg Lys Arg Arg Gly Ala
            20                  25                  30

Ala Ser Gln Ala Arg Gly Ser Asp Ser Glu Asp Gly Glu Phe Glu Ile
        35                  40                  45

Gln Ala Glu Asp Asp Ala Arg Ala Arg Lys Leu Gly Pro Gly Arg Pro
    50                  55                  60

Leu Pro Thr Phe Pro Thr Ser Glu Cys Thr Ser Asp Val Glu Pro Asp
65                  70                  75                  80

Thr Arg Glu Met Val Arg Ala Gln Asn Lys Lys Lys Lys Lys Ser Gly
                85                  90                  95

Gly Phe Gln Ser Met Gly Leu Ser Tyr Pro Val Phe Lys Gly Ile Met
            100                 105                 110

Lys Lys Gly Tyr Lys Val Pro Thr Pro Ile Gln Arg Lys Thr Ile Pro
        115                 120                 125

Val Ile Leu Asp Gly Lys Asp Val Val Ala Met Ala Arg Thr Gly Ser
    130                 135                 140

Gly Lys Thr Ala Cys Phe Leu Leu Pro Met Phe Glu Arg Leu Lys Thr
145                 150                 155                 160

His Ser Ala Gln Thr Gly Ala Arg Ala Leu Ile Leu Ser Pro Thr Arg
                165                 170                 175

Glu Leu Ala Leu Gln Thr Leu Lys Phe Thr Lys Glu Leu Gly Lys Phe
            180                 185                 190

Thr Gly Leu Lys Thr Ala Leu Ile Leu Gly Gly Asp Arg Met Glu Asp
        195                 200                 205

Gln Phe Ala Ala Leu His Glu Asn Pro Asp Ile Ile Ile Ala Thr Pro

210                 215                 220

Gly Arg Leu Val His Val Ala Val Glu Met Ser Leu Lys Leu Gln Ser
225                 230                 235                 240

Val Glu Tyr Val Val Phe Asp Glu Ala Asp Arg Leu Phe Glu Met Gly
                245                 250                 255

Phe Ala Glu Gln Leu Gln Glu Ile Ile Ala Arg Leu Pro Gly Gly His
            260                 265                 270

Gln Thr Val Leu Phe Ser Ala Thr Leu Pro Lys Leu Leu Val Glu Phe
        275                 280                 285

Ala Arg Ala Gly Leu Thr Glu Pro Val Leu Ile Arg Leu Asp Val Asp
    290                 295                 300

Thr Lys Leu Asn Glu Gln Leu Lys Thr Ser Phe Phe Leu Val Arg Glu
305                 310                 315                 320

Asp Thr Lys Ala Ala Val Leu Leu His Leu Leu His Asn Val Val Arg
                325                 330                 335

Pro Gln Asp Gln Thr Val Val Phe Val Ala Thr Lys His His Ala Glu
            340                 345                 350

Tyr Leu Thr Glu Leu Leu Thr Thr Gln Arg Val Ser Cys Ala His Ile
        355                 360                 365

Tyr Ser Ala Leu Asp Pro Thr Ala Arg Lys Ile Asn Leu Ala Lys Phe
    370                 375                 380

Thr Leu Gly Lys Cys Ser Thr Leu Ile Val Thr Asp Leu Ala Ala Arg
385                 390                 395                 400

Gly Leu Asp Ile Pro Leu Leu Asp Asn Val Ile Asn Tyr Ser Phe Pro
                405                 410                 415

Ala Lys Gly Lys Leu Phe Leu His Arg Val Gly Arg Val Ala Arg Ala
            420                 425                 430

Gly Arg Ser Gly Thr Ala Tyr Ser Leu Val Ala Pro Asp Glu Ile Pro
        435                 440                 445

Tyr Leu Leu Asp Leu His Leu Phe Leu Gly Arg Ser Leu Thr Leu Ala
    450                 455                 460

Arg Pro Leu Lys Glu Pro Ser Gly Val Ala Gly Val Asp Gly Met Leu
465                 470                 475                 480

Gly Arg Val Pro Gln Ser Val Val Asp Glu Glu Asp Ser Gly Leu Gln
                485                 490                 495

Ser Thr Leu Glu Ala Ser Leu Glu Leu Arg Gly Leu Ala Arg Val Ala
            500                 505                 510

Asp Asn Ala Gln Gln Gln Tyr Val Arg Ser Arg Pro Ala Pro Ser Pro
        515                 520                 525

Glu Ser Ile Lys Arg Ala Lys Glu Met Asp Leu Val Gly Leu Gly Leu
    530                 535                 540

His Pro Leu Phe Ser Ser Arg Phe Glu Glu Glu Glu Leu Gln Arg Leu
545                 550                 555                 560

Arg Leu Val Asp Ser Ile Lys Asn Tyr Arg Ser Arg Ala Thr Ile Phe
                565                 570                 575

Glu Ile Asn Ala Ser Ser Arg Asp Leu Cys Ser Gln Val Met Arg Ala
            580                 585                 590

Lys Arg Gln Lys Asp Arg Lys Ala Ile Ala Arg Phe Gln Gln Gly Gln
        595                 600                 605

Gln Gly Arg Gln Glu Gln Gln Glu Gly Pro Val Gly Pro Ala Pro Ser
    610                 615                 620

Arg Pro Ala Leu Gln Glu Lys Gln Pro Glu Lys Glu Glu Glu Glu Glu
625                 630                 635                 640

Ala Gly Glu Ser Val Glu Asp Ile Phe Ser Glu Val Val Gly Arg Lys
                645                 650                 655

Arg Gln Arg Ser Gly Pro Asn Arg Gly Ala Lys Arg Arg Arg Glu Glu
            660                 665                 670

Ala Arg Gln Arg Asp Gln Glu Phe Tyr Ile Pro Tyr Arg Pro Lys Asp
        675                 680                 685

Phe Asp Ser Glu Arg Gly Leu Ser Ile Ser Gly Glu Gly Gly Ala Phe
    690                 695                 700

Glu Gln Gln Ala Ala Gly Ala Val Leu Asp Leu Met Gly Asp Glu Ala
705                 710                 715                 720

Gln Asn Leu Thr Arg Gly Arg Gln Gln Leu Lys Trp Asp Arg Lys Lys
                725                 730                 735

Lys Arg Phe Val Gly Gln Ser Gly Gln Glu Asp Lys Lys Lys Ile Lys
            740                 745                 750

Thr Glu Ser Gly Arg Tyr Ile Ser Ser Ser Tyr Lys Arg Asp Leu Tyr
        755                 760                 765

Gln Lys Trp Lys Gln Lys Gln Lys Ile Asp Asp Arg Asp Ser Asp Glu
    770                 775                 780

Glu Gly Ala Ser Asp Arg Arg Gly Pro Glu Arg Arg Gly Gly Lys Arg
785                 790                 795                 800

Asp Arg Gly Gln Ala Gly Ala Ser Arg Pro His Ala Pro Gly Thr Pro
                805                 810                 815

Ala Gly Arg Val Arg Pro Glu Leu Lys Thr Lys Gln Gln Ile Leu Lys
            820                 825                 830

Gln Arg Arg Arg Ala Gln Lys Leu His Phe Leu Gln Arg Gly Gly Leu
        835                 840                 845

Lys Gln Leu Ser Ala Arg Asn Arg Arg Arg Val Gln Glu Leu Gln Gln
    850                 855                 860

Gly Ala Phe Gly Arg Gly Ala Arg Ser Lys Lys Gly Lys Met Arg Lys
865                 870                 875                 880

Arg Met

<210>  10
<211>  249
<212>  PRT
<213>  Homo sapiens

<400>  10

Met Ala Ala Gly Met Tyr Leu Glu His Tyr Leu Asp Ser Ile Glu Asn
1               5                   10                  15

Leu Pro Phe Glu Leu Gln Arg Asn Phe Gln Leu Met Arg Asp Leu Asp
            20                  25                  30

Gln Arg Thr Glu Asp Leu Lys Ala Glu Ile Asp Lys Leu Ala Thr Glu
        35                  40                  45

Tyr Met Ser Ser Ala Arg Ser Leu Ser Ser Glu Glu Lys Leu Ala Leu
    50                  55                  60

Leu Lys Gln Ile Gln Glu Ala Tyr Gly Lys Cys Lys Glu Phe Gly Asp
65                  70                  75                  80

Asp Lys Val Gln Leu Ala Met Gln Thr Tyr Glu Met Val Asp Lys His
                85                  90                  95

Ile Arg Arg Leu Asp Thr Asp Leu Ala Arg Phe Glu Ala Asp Leu Lys
            100                 105                 110

Glu Lys Gln Ile Glu Ser Ser Asp Tyr Asp Ser Ser Ser Ser Lys Gly
        115                 120                 125

Lys Lys Lys Gly Arg Thr Gln Lys Glu Lys Lys Ala Ala Arg Ala Arg
    130                 135                 140

Ser Lys Gly Lys Asn Ser Asp Glu Glu Ala Pro Lys Thr Ala Gln Lys
145                 150                 155                 160

Lys Leu Lys Leu Val Arg Thr Ser Pro Glu Tyr Gly Met Pro Ser Val
                165                 170                 175

Thr Phe Gly Ser Val His Pro Ser Asp Val Leu Asp Met Pro Val Asp
            180                 185                 190

Pro Asn Glu Pro Thr Tyr Cys Leu Cys His Gln Val Ser Tyr Gly Glu
        195                 200                 205

Met Ile Gly Cys Asp Asn Pro Asp Cys Ser Ile Glu Trp Phe His Phe
    210                 215                 220

Ala Cys Val Gly Leu Thr Thr Lys Pro Arg Gly Lys Trp Phe Cys Pro
225                 230                 235                 240

Arg Cys Ser Gln Glu Arg Lys Lys Lys
                245

<210>  11
<211>  156
<212>  PRT
<213>  Homo sapiens

<400>  11

Met Gln Ile Phe Val Lys Thr Leu Met Gly Lys Thr Ile Thr Leu Glu
1               5                   10                  15

Val Glu Leu Ser Asp Thr Ile Asp Asn Val Lys Ala Lys Ile Gln Asp
            20                  25                  30

Lys Glu Gly Ile Pro Pro Asp Gln Gln Arg Leu Ile Phe Ala Gly Lys
        35                  40                  45

Gln Leu Glu Asp Gly Arg Thr Leu Ser Asp Tyr Asn Ile Gln Lys Glu
    50                  55                  60

Ser Thr Leu His Leu Val Leu Arg Leu Arg Gly Gly Ala Lys Lys Arg
65                  70                  75                  80

Lys Lys Lys Ser Tyr Thr Thr Pro Arg Lys Asn Lys His Lys Arg Lys
                85                  90                  95

Lys Val Lys Leu Ala Leu Leu Lys Tyr Tyr Lys Val Asp Glu Asn Gly
            100                 105                 110

Lys Ile Ser Cys Leu His Arg Glu Cys Pro Ser Asp Glu Cys Gly Ala
        115                 120                 125

Gly Val Phe Met Ala Ser His Phe Asp Arg His Tyr Cys Gly Lys Cys
    130                 135                 140

Cys Leu Thr Tyr Cys Phe Asn Lys Pro Glu Asp Lys
145                 150                 155

<210>  12
<211>  859
<212>  PRT
<213>  Homo sapiens

<400>  12

Met Lys Leu Lys Asp Thr Lys Ser Arg Pro Lys Gln Ser Ser Cys Gly
1               5                   10                  15

Lys Phe Gln Thr Lys Gly Ile Lys Val Val Gly Lys Trp Lys Glu Val
            20                  25                  30

Lys Ile Asp Pro Asn Met Phe Ala Asp Gly Gln Met Asp Asp Leu Val
        35                  40                  45

Cys Phe Glu Glu Leu Thr Asp Tyr Gln Leu Val Ser Pro Ala Lys Asn
    50                  55                  60

Pro Ser Ser Leu Phe Ser Lys Glu Ala Pro Lys Arg Lys Ala Gln Ala
65                  70                  75                  80

Val Ser Glu Glu Glu Glu Glu Glu Glu Gly Lys Ser Ser Ser Pro Lys
                85                  90                  95

Lys Lys Ile Lys Leu Lys Lys Ser Lys Asn Val Ala Thr Glu Gly Thr
            100                 105                 110

Ser Thr Gln Lys Glu Phe Glu Val Lys Asp Pro Glu Leu Glu Ala Gln
        115                 120                 125

Gly Asp Asp Met Val Cys Asp Asp Pro Glu Ala Gly Glu Met Thr Ser
    130                 135                 140

Glu Asn Leu Val Gln Thr Ala Pro Lys Lys Lys Lys Asn Lys Gly Lys
145                 150                 155                 160

Lys Gly Leu Glu Pro Ser Gln Ser Thr Ala Ala Lys Val Pro Lys Lys
                165                 170                 175

Ala Lys Thr Trp Ile Pro Glu Val His Asp Gln Lys Ala Asp Val Ser
            180                 185                 190

Ala Trp Lys Asp Leu Phe Val Pro Arg Pro Val Leu Arg Ala Leu Ser
        195                 200                 205

Phe Leu Gly Phe Ser Ala Pro Thr Pro Ile Gln Ala Leu Thr Leu Ala
    210                 215                 220

Pro Ala Ile Arg Asp Lys Leu Asp Ile Leu Gly Ala Ala Glu Thr Gly
225                 230                 235                 240

Ser Gly Lys Thr Leu Ala Phe Ala Ile Pro Met Ile His Ala Val Leu
                245                 250                 255

Gln Trp Gln Lys Arg Asn Ala Ala Pro Pro Pro Ser Asn Thr Glu Ala
            260                 265                 270

Pro Pro Gly Glu Thr Arg Thr Glu Ala Gly Ala Glu Thr Arg Ser Pro
        275                 280                 285

Gly Lys Ala Glu Ala Glu Ser Asp Ala Leu Pro Asp Asp Thr Val Ile
    290                 295                 300

Glu Ser Glu Ala Leu Pro Ser Asp Ile Ala Ala Glu Ala Arg Ala Lys
305                 310                 315                 320

Thr Gly Gly Thr Val Ser Asp Gln Ala Leu Leu Phe Gly Asp Asp Asp
                325                 330                 335

Ala Gly Glu Gly Pro Ser Ser Leu Ile Arg Glu Lys Pro Val Pro Lys
            340                 345                 350

Gln Asn Glu Asn Glu Glu Glu Asn Leu Asp Lys Glu Gln Thr Gly Asn
        355                 360                 365

Leu Lys Gln Glu Leu Asp Asp Lys Ser Ala Thr Cys Lys Ala Tyr Pro
    370                 375                 380

Lys Arg Pro Leu Leu Gly Leu Val Leu Thr Pro Thr Arg Glu Leu Ala
385                 390                 395                 400

Val Gln Val Lys Gln His Ile Asp Ala Val Ala Arg Phe Thr Gly Ile
                405                 410                 415

Lys Thr Ala Ile Leu Val Gly Gly Met Ser Thr Gln Lys Gln Gln Arg
            420                 425                 430

Met Leu Asn Arg Arg Pro Glu Ile Val Val Ala Thr Pro Gly Arg Leu
        435                 440                 445

Trp Glu Leu Ile Lys Glu Lys His Tyr His Leu Arg Asn Leu Arg Gln
    450                 455                 460

Leu Arg Cys Leu Val Val Asp Glu Ala Asp Arg Met Val Glu Lys Gly
465                 470                 475                 480

His Phe Ala Glu Leu Ser Gln Leu Leu Glu Met Leu Asn Asp Ser Gln
                485                 490                 495

Tyr Asn Pro Lys Arg Gln Thr Leu Val Phe Ser Ala Thr Leu Thr Leu
            500                 505                 510

Val His Gln Ala Pro Ala Arg Ile Leu His Lys Lys His Thr Lys Lys
        515                 520                 525

Met Asp Lys Thr Ala Lys Leu Asp Leu Leu Met Gln Lys Ile Gly Met
    530                 535                 540

Arg Gly Lys Pro Lys Val Ile Asp Leu Thr Arg Asn Glu Ala Thr Val
545                 550                 555                 560

Glu Thr Leu Thr Glu Thr Lys Ile His Cys Glu Thr Asp Glu Lys Asp
                565                 570                 575

Phe Tyr Leu Tyr Tyr Phe Leu Met Gln Tyr Pro Gly Arg Ser Leu Val
            580                 585                 590

Phe Ala Asn Ser Ile Ser Cys Ile Lys Arg Leu Ser Gly Leu Leu Lys
        595                 600                 605

Val Leu Asp Ile Met Pro Leu Thr Leu His Ala Cys Met His Gln Lys
    610                 615                 620

Gln Arg Leu Arg Asn Leu Glu Gln Phe Ala Arg Leu Glu Asp Cys Val
625                 630                 635                 640

Leu Leu Ala Thr Asp Val Ala Ala Arg Gly Leu Asp Ile Pro Lys Val
                645                 650                 655

Gln His Val Ile His Tyr Gln Val Pro Arg Thr Ser Glu Ile Tyr Val
            660                 665                 670

His Arg Ser Gly Arg Thr Ala Arg Ala Thr Asn Glu Gly Leu Ser Leu
        675                 680                 685

Met Leu Ile Gly Pro Glu Asp Val Ile Asn Phe Lys Lys Ile Tyr Lys
    690                 695                 700

Thr Leu Lys Lys Asp Glu Asp Ile Pro Leu Phe Pro Val Gln Thr Lys
705                 710                 715                 720

Tyr Met Asp Val Val Lys Glu Arg Ile Arg Leu Ala Arg Gln Ile Glu

725                 730                 735

Lys Ser Glu Tyr Arg Asn Phe Gln Ala Cys Leu His Asn Ser Trp Ile
            740                 745                 750

Glu Gln Ala Ala Ala Ala Leu Glu Ile Glu Leu Glu Glu Asp Met Tyr
        755                 760                 765

Lys Gly Gly Lys Ala Asp Gln Gln Glu Glu Arg Arg Arg Gln Lys Gln
    770                 775                 780

Met Lys Val Leu Lys Lys Glu Leu Arg His Leu Leu Ser Gln Pro Leu
785                 790                 795                 800

Phe Thr Glu Ser Gln Lys Thr Lys Tyr Pro Thr Gln Ser Gly Lys Pro
                805                 810                 815

Pro Leu Leu Val Ser Ala Pro Ser Lys Ser Glu Ser Ala Leu Ser Cys
            820                 825                 830

Leu Ser Lys Gln Lys Lys Lys Lys Thr Lys Lys Pro Lys Glu Pro Gln
        835                 840                 845

Pro Glu Gln Pro Gln Pro Ser Thr Ser Ala Asn
    850                 855

<210>  13
<211>  126
<212>  PRT
<213>  Homo sapiens

<400>  13

Met Ala Ala Gly Pro Glu Pro Pro Glu Trp Glu Pro Arg Trp Arg Lys
1               5                   10                  15

Ala Leu Arg Gly Lys Glu Asn Lys Gly Ser Val Glu Ile Met Arg Lys
            20                  25                  30

Asp Leu Asn Asp Ala Arg Asp Leu His Gly Gln Ala Glu Ser Ala Ala
        35                  40                  45

Ala Val Trp Lys Gly His Val Met Asp Arg Arg Lys Lys Ala Leu Thr
    50                  55                  60

Asp Tyr Lys Lys Leu Arg Ala Phe Phe Val Glu Glu Glu Glu His Phe
65                  70                  75                  80

Leu Gln Glu Ala Glu Lys Glu Glu Gly Leu Pro Glu Asp Glu Leu Ala
                85                  90                  95

Asp Pro Thr Glu Arg Phe Arg Ser Leu Leu Gln Ala Val Ser Glu Leu
            100                 105                 110

Glu Lys Lys His Arg Asn Leu Gly Leu Ser Met Leu Leu Gln
        115                 120                 125

<210>  14
<211>  710
<212>  PRT
<213>  Homo sapiens

<400>  14

Met Ser Val Pro Ser Ser Leu Ser Gln Ser Ala Ile Asn Ala Asn Ser
1               5                   10                  15

His Gly Gly Pro Ala Leu Ser Leu Pro Leu Pro Leu His Ala Ala His
            20                  25                  30

Asn Gln Leu Leu Asn Ala Lys Leu Gln Ala Thr Ala Val Gly Pro Lys
        35                  40                  45

Asp Leu Arg Ser Ala Met Gly Glu Gly Gly Gly Pro Glu Pro Gly Pro
    50                  55                  60

Ala Asn Ala Lys Trp Leu Lys Glu Gly Gln Asn Gln Leu Arg Arg Ala
65                  70                  75                  80

Ala Thr Ala His Arg Asp Gln Asn Arg Asn Val Thr Leu Thr Leu Ala
                85                  90                  95

Glu Glu Ala Ser Gln Glu Pro Glu Met Ala Pro Leu Gly Pro Lys Gly

            100                 105                 110

Leu Ile His Leu Tyr Ser Glu Leu Glu Leu Ser Ala His Asn Ala Ala
        115                 120                 125

Asn Arg Gly Leu Arg Gly Pro Gly Leu Ile Ile Ser Thr Gln Glu Gln
    130                 135                 140

Gly Pro Asp Glu Gly Glu Glu Lys Ala Ala Gly Glu Ala Glu Glu Glu
145                 150                 155                 160

Glu Glu Asp Asp Asp Asp Glu Glu Glu Glu Glu Asp Leu Ser Ser Pro
                165                 170                 175

Pro Gly Leu Pro Glu Pro Leu Glu Ser Val Glu Ala Pro Pro Arg Pro
            180                 185                 190

Gln Ala Leu Thr Asp Gly Pro Arg Glu His Ser Lys Ser Ala Ser Leu
        195                 200                 205

Leu Phe Gly Met Arg Asn Ser Ala Ala Ser Asp Glu Asp Ser Ser Trp
    210                 215                 220

Ala Thr Leu Ser Gln Gly Ser Pro Ser Tyr Gly Ser Pro Glu Asp Thr
225                 230                 235                 240

Asp Ser Phe Trp Asn Pro Asn Ala Phe Glu Thr Asp Ser Asp Leu Pro
                245                 250                 255

Ala Gly Trp Met Arg Val Gln Asp Thr Ser Gly Thr Tyr Tyr Trp His
            260                 265                 270

Ile Pro Thr Gly Thr Thr Gln Trp Glu Pro Pro Gly Arg Ala Ser Pro
        275                 280                 285

Ser Gln Gly Ser Ser Pro Gln Glu Glu Ser Gln Leu Thr Trp Thr Gly
    290                 295                 300

Phe Ala His Gly Glu Gly Phe Glu Asp Gly Glu Phe Trp Lys Asp Glu
305                 310                 315                 320

Pro Ser Asp Glu Ala Pro Met Glu Leu Gly Leu Lys Glu Pro Glu Glu
                325                 330                 335

Gly Thr Leu Thr Phe Pro Ala Gln Ser Leu Ser Pro Glu Pro Leu Pro
            340                 345                 350

Gln Glu Glu Glu Lys Leu Pro Pro Arg Asn Thr Asn Pro Gly Ile Lys
        355                 360                 365

Cys Phe Ala Val Arg Ser Leu Gly Trp Val Glu Met Thr Glu Glu Glu
    370                 375                 380

Leu Ala Pro Gly Arg Ser Ser Val Ala Val Asn Asn Cys Ile Arg Gln
385                 390                 395                 400

Leu Ser Tyr His Lys Asn Asn Leu His Asp Pro Met Ser Gly Gly Trp
                405                 410                 415

Gly Glu Gly Lys Asp Leu Leu Leu Gln Leu Glu Asp Glu Thr Leu Lys
            420                 425                 430

Leu Val Glu Pro Gln Ser Gln Ala Leu Leu His Ala Gln Pro Ile Ile
        435                 440                 445

Ser Ile Arg Val Trp Gly Val Gly Arg Asp Ser Gly Arg Glu Arg Asp
    450                 455                 460

Phe Ala Tyr Val Ala Arg Asp Lys Leu Thr Gln Met Leu Lys Cys His
465                 470                 475                 480

Val Phe Arg Cys Glu Ala Pro Ala Lys Asn Ile Ala Thr Ser Leu His
                485                 490                 495

Glu Ile Cys Ser Lys Ile Met Ala Glu Arg Arg Asn Ala Arg Cys Leu
            500                 505                 510

Val Asn Gly Leu Ser Leu Asp His Ser Lys Leu Val Asp Val Pro Phe
        515                 520                 525

Gln Val Glu Phe Pro Ala Pro Lys Asn Glu Leu Val Gln Lys Phe Gln
    530                 535                 540

Val Tyr Tyr Leu Gly Asn Val Pro Val Ala Lys Pro Val Gly Val Asp
545                 550                 555                 560

Val Ile Asn Gly Ala Leu Glu Ser Val Leu Ser Ser Ser Ser Arg Glu
                565                 570                 575

Gln Trp Thr Pro Ser His Val Ser Val Ala Pro Ala Thr Leu Thr Ile
            580                 585                 590

Leu His Gln Gln Thr Glu Ala Val Leu Gly Glu Cys Arg Val Arg Phe
        595                 600                 605

Leu Ser Phe Leu Ala Val Gly Arg Asp Val His Thr Phe Ala Phe Ile
    610                 615                 620

Met Ala Ala Gly Pro Ala Ser Phe Cys Cys His Met Phe Trp Cys Glu
625                 630                 635                 640

Pro Asn Ala Ala Ser Leu Ser Glu Ala Val Gln Ala Ala Cys Met Leu
                645                 650                 655

Arg Tyr Gln Lys Cys Leu Asp Ala Arg Ser Gln Ala Ser Thr Ser Cys
            660                 665                 670

Leu Pro Ala Pro Pro Ala Glu Ser Val Ala Arg Arg Val Gly Trp Thr
        675                 680                 685

Val Arg Arg Gly Val Gln Ser Leu Trp Gly Ser Leu Lys Pro Lys Arg
    690                 695                 700

Leu Gly Ala His Thr Pro
705                 710

<210>  15
<211>  316
<212>  PRT
<213>  Homo sapiens

<400>  15

Met Ala Ser Thr Ser Arg Leu Asp Ala Leu Pro Arg Val Thr Cys Pro
1               5                   10                  15

Asn His Pro Asp Ala Ile Leu Val Glu Asp Tyr Arg Ala Gly Asp Met
            20                  25                  30

Ile Cys Pro Glu Cys Gly Leu Val Val Gly Asp Arg Val Ile Asp Val
        35                  40                  45

Gly Ser Glu Trp Arg Thr Phe Ser Asn Asp Lys Ala Thr Lys Asp Pro
    50                  55                  60

Ser Arg Val Gly Asp Ser Gln Asn Pro Leu Leu Ser Asp Gly Asp Leu
65                  70                  75                  80

Ser Thr Met Ile Gly Lys Gly Thr Gly Ala Ala Ser Phe Asp Glu Phe
                85                  90                  95

Gly Asn Ser Lys Tyr Gln Asn Arg Arg Thr Met Ser Ser Ser Asp Arg
            100                 105                 110

Ala Met Met Asn Ala Phe Lys Glu Ile Thr Thr Met Ala Asp Arg Ile
        115                 120                 125

Asn Leu Pro Arg Asn Ile Val Asp Arg Thr Asn Asn Leu Phe Lys Gln
    130                 135                 140

Val Tyr Glu Gln Lys Ser Leu Lys Gly Arg Ala Asn Asp Ala Ile Ala
145                 150                 155                 160

Ser Ala Cys Leu Tyr Ile Ala Cys Arg Gln Glu Gly Val Pro Arg Thr
                165                 170                 175

Phe Lys Glu Ile Cys Ala Val Ser Arg Ile Ser Lys Lys Glu Ile Gly
            180                 185                 190

Arg Cys Phe Lys Leu Ile Leu Lys Ala Leu Glu Thr Ser Val Asp Leu
        195                 200                 205

Ile Thr Thr Gly Asp Phe Met Ser Arg Phe Cys Ser Asn Leu Cys Leu
    210                 215                 220

Pro Lys Gln Val Gln Met Ala Ala Thr His Ile Ala Arg Lys Ala Val
225                 230                 235                 240

Glu Leu Asp Leu Val Pro Gly Arg Ser Pro Ile Ser Val Ala Ala Ala
                245                 250                 255

Ala Ile Tyr Met Ala Ser Gln Ala Ser Ala Glu Lys Arg Thr Gln Lys
            260                 265                 270

Glu Ile Gly Asp Ile Ala Gly Val Ala Asp Val Thr Ile Arg Gln Ser
        275                 280                 285

Tyr Arg Leu Ile Tyr Pro Arg Ala Pro Asp Leu Phe Pro Thr Asp Phe
    290                 295                 300

Lys Phe Asp Thr Pro Val Asp Lys Leu Pro Gln Leu
305                 310                 315

<210>  16
<211>  123
<212>  PRT
<213>  Homo sapiens

<400>  16

<210>  17
<211>  558
<212>  PRT
<213>  Homo sapiens

<400>  17

<210>  18
<211>  599
<212>  PRT
<213>  Homo sapiens

<400>  18

Met Gly Pro Lys Arg Arg Gln Leu Thr Phe Arg Glu Lys Ser Arg Ile
1               5                   10                  15

Ile Gln Glu Val Glu Glu Asn Pro Asp Leu Arg Lys Gly Glu Ile Ala
            20                  25                  30

Arg Arg Phe Asn Ile Pro Pro Ser Thr Leu Ser Thr Ile Leu Lys Asn
        35                  40                  45

Lys Arg Ala Ile Leu Ala Ser Glu Arg Lys Tyr Gly Val Ala Ser Thr
    50                  55                  60

Cys Arg Lys Thr Asn Lys Leu Ser Pro Tyr Asp Lys Leu Glu Gly Leu
65                  70                  75                  80

Leu Ile Ala Trp Phe Gln Gln Ile Arg Ala Ala Gly Leu Pro Val Lys
                85                  90                  95

Gly Ile Ile Leu Lys Glu Lys Ala Leu Arg Ile Ala Glu Glu Leu Gly
            100                 105                 110

Met Asp Asp Phe Thr Ala Ser Asn Gly Trp Leu Asp Arg Phe Arg Arg
        115                 120                 125

Arg His Gly Val Val Ser Cys Ser Gly Val Ala Arg Ala Arg Ala Arg
    130                 135                 140

Asn Ala Ala Pro Arg Thr Pro Ala Ala Pro Ala Ser Pro Ala Ala Val
145                 150                 155                 160

Pro Ser Glu Gly Ser Gly Gly Ser Thr Thr Gly Trp Arg Ala Arg Glu
                165                 170                 175

Glu Gln Pro Pro Ser Val Ala Glu Gly Tyr Ala Ser Gln Asp Val Phe
            180                 185                 190

Ser Ala Thr Glu Thr Ser Leu Trp Tyr Asp Phe Leu Pro Asp Gln Ala
        195                 200                 205

Ala Gly Leu Cys Gly Gly Asp Gly Arg Pro Arg Gln Ala Thr Gln Arg
    210                 215                 220

Leu Ser Val Leu Leu Cys Ala Asn Ala Asp Gly Ser Glu Lys Leu Pro
225                 230                 235                 240

Pro Leu Val Ala Gly Lys Ser Ala Lys Pro Arg Ala Gly Gln Ala Gly
                245                 250                 255

Leu Pro Cys Asp Tyr Thr Ala Asn Ser Lys Gly Gly Val Thr Thr Gln
            260                 265                 270

Ala Leu Ala Lys Tyr Leu Lys Ala Leu Asp Thr Arg Met Ala Ala Glu
        275                 280                 285

Ser Arg Arg Val Leu Leu Leu Ala Gly Arg Leu Ala Ala Gln Ser Leu
    290                 295                 300

Asp Thr Ser Gly Leu Arg His Val Gln Leu Ala Phe Phe Pro Pro Gly
305                 310                 315                 320

Thr Val His Pro Leu Glu Arg Gly Val Val Gln Gln Val Lys Gly His
                325                 330                 335

Tyr Arg Gln Ala Met Leu Leu Lys Ala Met Ala Ala Leu Glu Gly Gln
            340                 345                 350

Asp Pro Ser Gly Leu Gln Leu Gly Leu Thr Glu Ala Leu His Phe Val
        355                 360                 365

Ala Ala Ala Trp Gln Ala Val Glu Pro Ser Asp Ile Ala Ala Cys Phe
    370                 375                 380

Arg Glu Ala Gly Phe Gly Gly Gly Pro Asn Ala Thr Ile Thr Thr Ser
385                 390                 395                 400

Leu Lys Ser Glu Gly Glu Glu Glu Glu Glu Glu Glu Glu Glu Glu Glu
                405                 410                 415

Glu Glu Glu Gly Glu Gly Glu Glu Glu Glu Glu Glu Gly Glu Glu Glu
            420                 425                 430

Glu Glu Glu Gly Gly Glu Gly Glu Glu Leu Gly Glu Glu Glu Glu Val
        435                 440                 445

Glu Glu Glu Gly Asp Val Asp Ser Asp Glu Glu Glu Glu Glu Asp Glu
    450                 455                 460

Glu Ser Ser Ser Glu Gly Leu Glu Ala Glu Asp Trp Ala Gln Gly Val
465                 470                 475                 480

Val Glu Ala Gly Gly Ser Phe Gly Ala Tyr Gly Ala Gln Glu Glu Ala
                485                 490                 495

Gln Cys Pro Thr Leu His Phe Leu Glu Gly Gly Glu Asp Ser Asp Ser
            500                 505                 510

Asp Ser Glu Glu Glu Asp Asp Glu Glu Glu Asp Asp Glu Asp Glu Asp
        515                 520                 525

Asp Asp Asp Asp Glu Glu Asp Gly Asp Glu Val Pro Val Pro Ser Phe
    530                 535                 540

Gly Glu Ala Met Ala Tyr Phe Ala Met Val Lys Arg Tyr Leu Thr Ser
545                 550                 555                 560

Phe Pro Ile Asp Asp Arg Val Gln Ser His Ile Leu His Leu Glu His
                565                 570                 575

Asp Leu Val His Val Thr Arg Lys Asn His Ala Arg Gln Ala Gly Val
            580                 585                 590

Arg Gly Leu Gly His Gln Ser
        595